# EUROPEAN PATENT SPECIFICATION

(11) **EP 0 958 357 B1**
(45) Date of publication and mention of the grant of the patent: **23.11.2005**
(21) Application number: 97954761.9
(22) Date of filing: 30.12.1997
(51) Int. Cl.: C12N 15/09, C12Q 1/68, C07K 14/47, C12N 5/06, A01K 67/027, A61K 31/70, G01N 33/574

(54) **NOVEL METHOD FOR TESTING THE DIFFERENTIATION STATUS IN PANCREATIC CELLS OF A MAMMAL**
NEUES VERFAHREN ZUR ÜBERPRÜFUNG DES DIFFERENZIERUNGSZUSTANDS PANKREATISCHER ZELLEN EINES SÄUGETIERS
NOUVEAU PROCEDE POUR TESTER L'ETAT DE DIFFERENCIATION DES CELLULES PANCREATIQUES CHEZ LE MAMMIFERE

(30) Priority: 31.12.1996 US 778423
(43) Date of publication of application: 24.11.1999
(60) Divisional application: 02018275.4
(73) Proprietor: Max-Planck-Gesellschaft zur Förderung der Wissenschaften e.V., Berlin (DE)
(72) Inventor: SOSA-PINEDA, Beatriz, Memphis, TN 38120 (US); GRUSS, Peter, D-37085 Göttingen (DE)
(74) Representative: Vossius & Partner
(86) International application number: PCT/EP1997/007321
(87) International publication number: WO 1998/029566

(56) References cited:
- WO-A-94/03196
- TURQUE ET AL.: "PX-QNR/PAX-6, A PAIRED BOX-AND HOMEOBOX-CONTAINING GENE EXPRESSED IN NEURONS, IS ALSO EXPRESSED IN PANCREATIC ENDOCRINE CELLS" MOL.ENDOCRINOLOGY, vol. 8, no. 7, 1995, pages 929-938, XP002066644
- "THE PAX4 GENE IS ESSENTIAL FOR DIFFERENTIATION OF INSULIN-PRODUCING BETA CELLS IN THE MAMMALIAN PANCREAS" NATURE, vol. 386, March 1997, pages 399-402, XP002066645
- ST-ONGE ET AL.: "PAX6 IS REQUIRED FOR DIFFERENTIATION OF GLUCAGON-PRODUCING ALPHA-CELLS IN MOUSE PANCREAS" NATURE, vol. 387, May 1997, pages 406-409, XP002066646

## Description

### FIELD OF THE INVENTION

The present invention relates to methods for testing the differentiation status of pancreatic cells in a mammal; for instance, by ascertaining the level or status of Pax4 mRNA and/or protein in pancreatic cells (or pre-pancreatic cells) and comparing the level or status with the corresponding level or status in normal pancreatic (or pre-pancreatic) cells. This method provides a means for diagnosis or detection of diseases which arise from certain pancreatic cells, especially a means for diagnosis or detection of, for instance, diabetes, such as juvenile diabetes. The method can be performed in conjunction with ascertaining the level or status of Pax6 mRNA and/or protein in pancreatic cells (or pre-pancreatic cells) and comparing the level or status with the corresponding level or status in normal pancreatic (or pre-pancreatic) cells.

The data set forth below shows that deficiency in Pax4 expression is indicative of deficiency or failure in β-cell development and ergo insulin production (and thus diabetes such as juvenile diabetes). The invention thus relates to restoring Pax4 expression for treatment, prevention or delaying a pancreatic disease such as diabetes, e.g., juvenile diabetes; and ergo transgenic mammals having restored Pax4 expression by modification so as to comprise at least one first nucleic acid molecule having a sequence encoding a functional and expressible Pax4 protein and optionally a second nucleic acid sequence encoding a functional and expressible Pax6 protein. Alternatively or additionally, the invention relates to administration of Pax4 alone or with Pax6 and/or of an agent for stimulating expression of Pax4 or Pax4 and Pax6, for treatment, prevention or delaying a pancreatic disease such as diabetes, e.g., juvenile diabetes.

Since the data set forth below shows that deficiency in Pax4 expression is indicative of deficiency or failure in β-cell development, and ergo insulin production (and thus diabetes such as juvenile diabetes), the present invention also relates to transgenic mammals modified so as to comprise at least one inactivated Pax4 allele. This mammal has numerous utilities, including as a research model for pancreatic diseases such as juvenile diabetes; and therefore, presents a novel and valuable animal in the development of therapies, treatment, etc. for diseases caused by deficiency or failure of pancreatic cells. Accordingly, in this instance, the mammal is non-human, e.g., a laboratory animal such as a mouse or rat.

Further, since improper expression of Pax4 may also cause maladies such as tumors, the invention also relates to at least one inactivated Pax4 allele for treatment, prevention or the delay of a pancreatic disease caused by improper expression of Pax4, such as tumors. In this instance, the mammal to be treated can be a human, as the introduction into the mammal of the at least one inactivated Pax4 allele is for therapy. Alternatively or additionally, the invention relates to administration of an agent which inhibits Pax4 or Pax4 and Pax6 for treatment, prevention or delaying a pancreatic disease caused by improper expression of Pax4, such as tumors.

Several documents are cited throughout the text of this specification. Each of the documents cited herein (including manufacturer's specifications, instructions, etc.) are hereby incorporated herein by reference.

### BACKGROUND OF THE INVENTION

The pancreas is an essential organ possessing both an exocrine function involved in the delivery of enzymes into the digestive tract and an endocrine function by which various hormones are secreted into the blood stream. The exocrine function is assured by acinar and centroacinar cells that produce various digestive enzymes (amylase, proteases, nuclease, etc.) and intercalated duct cells that transport these enzymes in alkaline solution to the duodenum.

The functional unit of the endocrine pancreas is the islet of Langerhans which are scattered throughout the exocrine portion of the pancreas and are composed of four cell types: α-, β-, δ- and PP-cells, reviewed in Slack, Development 121 (1995), 1569-1580. β-cells produce insulin, represent the majority of the endocrine cells and form the core of the islets, while α-cells secrete glucagon and are located in the periphery. δ-cells and PP-cells are less numerous and respectively secrete somatostatin and a pancreatic polypeptide. Insulin and glucagon are key regulators of blood glucose levels. Insulin lowers blood glucose level by increasing the cellular uptake of glucose and its conversion to glycogen. Glucagon elevates blood glucose levels by intervening with the breakdown of liver glycogen. Common pancreatic disorders affecting endocrine function include diabetes mellitus and hormone secreting tumors.

All four endocrine cells are thought to originate from a common pluripotent precursor that is derived from the endoderm. Early during pancreatic development, these precursors co-express several hormones such as insulin and glucagon. In mouse, the α-cells are the first endocrine cells to differentiate at day 9.5 post-conception (p.c.), followed by the β- and δ-cells at day 14.5 p.c. and the PP-cells at postnatal day 1. Very little is known on the molecular and genetic factors involved in defining the lineage of the different endocrine cells. One of the few genes described so far is the homeobox gene Pdx1 which is expressed during the initial stages of pancreatic development and becomes restricted to the β-cells in adult islets (Guz et al., Development 121 (1995), 11-18). Homozygous mouse Pdx1 mutants fail to develop a pancreas and die a few days after birth (Jonsson et al., Nature 371 (1994), 606-609). Two members of the Pax gene family, Pax4 and Pax6, are also expressed in endocrine cells during pancreatic development. Until now, however, it was not known how in particular the Pax4 and the Pax6 gene affects pancreatic development.

A method for testing.for a variety of differentiation parameters in the pancreas was hitherto not available but is nevertheless highly desirable. Results obtainable by such a method have a significant impact on, e.g., the diagnosis and therapy of pancreas related diseases.

### SUMMARY OF THE INVENTION

The present invention relates to a novel in vitro method for testing the differentiation status of pancreatic cells in mammals. The present invention further relates to applications in the medical field that directly arise from the method of the invention. Additionally, the present invention relates to non-human transgenic mammals comprising at least one inactivated Pax4 gene and optionally at least one inactivated Pax6 gene.

### DETAILED DESCRIPTION OF THE INVENTION

Accordingly, a technical problem underlying the present invention was to provide such a method for monitoring the differentiation status of pancreatic cells. The solution to said technical problem is achieved by the embodiments characterized in the claims.

Thus, the present invention relates to an in vitro method for testing the differentiation status in pancreatic cells of a mammal comprising.
(a) determining the level or status of Pax4 mRNA in pancreatic cells of said mammal; and/or
(b) determining the level or status of Pax4 protein in pancreatic cells of said mammal; and
(c) comparing said level or status of Pax4 mRNA and/or Pax4 protein with the corresponding level in normal pancreatic cells,
wherein said determination and comparison of the status of Pax4 mRNA and/or protein denotes the determination and comparison whether said mRNA or protein bears a mutation, deletion, or a post-translational modification which would affect the overall activity of the gene when compared to the wild-type normal gene product.

In connection with the present invention, the term "level" denotes the amount of mRNA or protein produced. The term "status" includes the options that the gene, mRNA, protein or a transcription control element, e.g. promoter/enhancer sequence may bear a mutation, deletion or any other modifications which would affect the overall activity of the gene when compared to the wild-type normal gene product. Included in this term are post-translational modifications of the protein.

The method of the present invention allows for the first time a detailed study of the development of different cell types, i.e. α-, β-, δ- and pp-cells in the pancreatic tissue. As is demonstrated by the appended examples, the Pax4 gene, optionally in conjunction with the Pax6 gene is involved in the early steps of pancreatic development. This surprising result allows the monitoring of cell fate as well as the investigation of the development of diseases which arise from certain cell types contained in the pancreas. The results presented in accordance with the present invention furthermore allow the conclusion that Pax4 and/or Pax6 are master regulators for specific pancreatic cells. Thus, Pax4 appears to be a master regulator of β-cells. Pax4 is also expressed in non-pancreatic cells. Pax4 expression is detected in a subset of cells found in the developing spinal cord, Therefore, Pax4 may likewise play a role in the differentiation status of these cells.

Accordingly, the method of the present invention provides a means for diagnosis or detection of diseases which arise from certain pancreatic cells, especially a means for early detection or diagnosis; for instance, detection or diagnosis of diabetes, such as juvenile diabetes; and, such detection or diagnosis can be pre- or post- natal.

In a preferred embodiment, the in vitro method of the present invention further comprises
(d) determining the level or status of Pax6. mRNA in pancreatic cells of said mammal; and/or
(e) determining the level or status of Pax6 protein in pancreatic cells of said mammal; and
(f) comparing said level or status of Pax6 mRNA and/or Pax6 protein with the corresponding level in normal pancreatic cells,
wherein said determination and comparison of the status of Pax6 mRNA and/or Pax6 protein denotes the determination and comparison whether said mRNA or protein bears a mutation, deletion, or a post-translational modification which would affect the overall activity of the gene when compared to the wild-type normal gene product.

This embodiment of the present invention allows the Study of the synergistic effects of the Pax4 and the Pax6 gene products. It is expected that the analysis of said synergistic effect provides deeper insights into the regulation of cell specific development in the pancreas.

From said deeper insight the development of diagnostic and pharmaceutical compositions-related to pancreas-specific diseases will greatly benefit.

In a further preferred embodiment of the in vitro method of the invention, said mammal is in the
(i) embryonic;
(ii) newborn; or
(iii) adult stage.

As has been shown in accordance with the present invention, the Pax4 gene, preferably in conjunction with the Pax6 gene, is expressed at a different level and at different stages of mammalian pancreas development. The specific analysis of the developmental stage of pancreatic cells at the embryonic, newborn or adult stage will provide further insights into, e.g., specific disease states associated with the respective stages. For example, it is expected that the etiology of, e.g., juvenile diabetes will be elucidated by applying the method of the present invention, as well as by employing the transgenic mammals (non-human) according to the invention (discussed infra; see also the examples). Upon the basis of this knowledge, new pharmaceutical active drugs, e.g. against juvenile diabetes, will be developed and tested.

The method of the invention can be applied to a variety of mammals, depending on the purpose of the investigation. Thus, in a preferred embodiment, the mammal is a mouse. This embodiment is particularly useful for basic research to understand more clearly the functional interrelationship of different proteins which regulate the development of the pancreas. In a further embodiment the mammal is a human. In this embodiment, preferably diagnostic and therapeutic applications are envisaged.

The method of the invention, steps (a) and optionally (d) and/or (b) and optionally (e) are carried out in vitro.

The advantages of this embodiment would be expected to lie primarily in diagnostic applications and, again, in basic research.

In a further preferred embodiment of the method of the invention, said determination in step (a) and optionally in step (d) is effected by employing
(i) a nucleic acid sequence corresponding to at least a part of the Pax4 gene and preferably encoding at least part of the Pax4 protein and optionally a second nucleic acid sequence corresponding to at least a part of the Pax6 gene and preferably encoding at least part of the Pax6 protein;
(ii) a nucleic acid sequence complementary to the nucleic acid sequence (s) of (i); or
(iii) a primer or a primer pair hybridizing to the nucleic acid sequence(s) of (i) or (ii).

In accordance with this embodiment of the present invention, the method of testing the differentiation status can be effected by using a nucleic acid molecule encoding the Pax4 gene and/or the Pax6 gene or a part thereof, e.g., in the form of a Southern or Northern blot or *in situ* analysis. Said nucleic acid sequence may hybridize to a coding region of either of the genes or to a non-coding region. In the case that a complementary sequence in accordance' with (ii) is employed in the method of the invention, said nucleic acid molecule can again be used in Northern blots. Additionally, said testing can be done in conjunction with an actual blocking, e.g., of the transcription of the gene and thus is expected to have therapeutic relevance. Furthermore, a primer or oligonucleotide can also be used for hybridizing to one of the above-mentioned Pax genes or corresponding mRNAs. The nucleic acids used for hybridization can, of course, be conveniently labeled by incorporating or attaching, e.g., a radioactive or other marker. Such markers are well known in the art. The labeling of said nucleic acid molecules can be effected by conventional methods.

Additionally, the presence or expression of Pax4 and optionally Pax6 can be monitored by using a primer pair that specifically hybridizes to either of the corresponding nucleic acid sequences and by carrying out a PCR reaction according to standard procedures.

Specific hybridization of the above mentioned probes or primers preferably occurs at stringent hybridization conditions. The term "stringent hybridization conditions" is well known in the art; see, for example, Sambrook et al., "Molecular Cloning, A Laboratory Manual" second ed., CSH Press, Cold Spring Harbor, 1989; "Nucleic Acid Hybridisation, A Practical Approach", Hames and Higgins eds., IRL Press, Oxford, 1985.

Further modifications of the above-mentioned embodiment of the invention can be easily devised by the person skilled in the art, without any undue experimentation from this disclosure.

An additional embodiment of the present invention relates to a method wherein said determination in step (b) and optionally of step (e) is effected by employing an antibody or fragment thereof that specifically binds to the Pax4 protein and optionally by employing a second antibody or fragment thereof which specifically binds to the Pax6 protein.

Antibodies or fragments thereof to the aforementioned protein can be obtained by using conventional methods which are described, e.g., in Harlow and Lane "Antibodies, A Laboratory Manual", CSH Press, Cold Spring Harbor, 1988. These antibodies may be monoclonal antibodies or comprised in polyclonal antisera or fragments thereof. The antibody used in the method of the invention may be labeled with detectable tags such as a histidine flags or a biotin molecule.

In a further preferred embodiment of the method of the present invention, said pancreatic cells are β-cells or δ-cells.

In accordance with the present invention, it was found that Pax4 is expressed in β-cells. Disruption of the Pax4 gene function therefore allows a close monitoring of the development of said cells in the pancreas. Since Pax6 is also expressed in both α- and β-cells, Pax6 expression may be required to establish a competent background for Pax4 to act.

Since both Pax4 and Pax6 are regulatory proteins required for proper differentiation of endocrine cells, this embodiment may allow a variety of conclusions with regard to the generation of diseases such as pancreatic tumors.

Accordingly, a further preferred embodiment relates to a method wherein said differentiation status is indicative of a malignancy or malignant potential of said pancreatic cell. Overexpression or absence of a functional Pax4 and optionally Pax6 product may induce normal endocrine cells to become cancerous. In accordance with this statement, allelic deletions in chromosome 7q in the vicinity of the Pax4 gene are observed in many pancreatic carcinomas (Alberto et al., Cancer Res. 56 (1996), 3808-3818). Pax4, and optionally Pax6, may also interact with other oncogenic factors.

Malignancy or malignant potential is used in accordance with this invention preferably but not exclusively in connection with pancreatic tumors such as insulinoma, glucagonomas, somatostatinomas and ductal cell adenocarcinomas.

The present invention relates in a further preferred embodiment to a method that further comprises
(a') prior to said testing removal of a solid pancreatic tumor from said mammal; and
(b') after said testing, at least partial elimination of the expression of the Pax4 and optionally the Pax6 gene in cells of said tumor, if said gene(s) is/are over-expressed or stimulation of expression of the Pax4 gene and optionally the Pax6 gene or introduction of a functional and expressible Pax4 gene and optionally a functional and expressible Pax6 gene into said cells if said gene(s) is/are under-expressed or not expressed; and
(b'') reintroducing the cells obtained as a product of step (b') into said mammal.

In this context and as used throughout this specification, "functional" Pax4 (Pax6) means a protein having part or all of the primary structural conformation of the Pax4 (Pax6) protein possessing the biological property of contributing to the development of endocrine cells into β-cells (a-cells and Langerhans cells), said protein being the product of procaryotic or eukaryotic expression of a Pax4 (Pax6) encoding DNA sequence and having an amino acid sequence comprising the amino acid sequence of SEQ ID No. 2 or 18 (SEQ ID No. 4) or any fragment or derivative thereof by way of amino acid deletion, substitution, insertion, addition and/or replacement of the amino acid sequence given in SEQ ID No. 2 or 18 (SEQ ID No. 4). Also comprised by the term "functional" Pax4 (Pax6) protein is the capability of said protein or part thereof to generate a specific immune response such as an antibody response.

This embodiment of the present invention is suited for therapy of tumors, in particular in humans. Therefore, it is envisaged that pancreatic tumor cells are monitored for the expression level of the Pax4 protein and optionally Pax6 protein. Detection of an over-expression of said protein(s) would allow the conclusion that said over-expression is interrelated to the generation or maintenance of the tumor. Accordingly, a step would be applied to reduce the expression level to normal levels. This can be done, for example, by at least partial elimination of the expression of the Pax4 gene by biological means, for example, by the use of ribozymes, antisense nucleic acid molecules or intracellular antibodies against either the Pax4 or Pax6 protein. Furthermore, pharmaceutical products may be developed that reduce the expression levels of Pax4. While it is presently unclear how Pax4 and optionally Pax6 are regulated in pancreatic tissue, it is possible that different developmental and hormonal factors determine the levels of activity of these genes. For example, small molecules are known to repress the expression of certain genes. It has been demonstrated that activin A, a member of the TGFβ superfamily, can downregulate Pax6 expression in the developing spinal cord (Pituello et al., Proc. Natl. Acad. Sci. USA 92 (1995), 6952-6956). Similar molecules may downregulate Pax4 or optionally Pax6 expression in the pancreas. On the other hand, lack of expression or under-expression may be remedied by a functional Pax4 gene and optionally a functional Pax6 gene which should both be expressible in the tumor cells.

Stimulation or induction of expression can be obtained again by the use of small molecules or other means, this time activating Pax4 gene expression. In this regard, it is important to note that the present invention envisages the possibilities that one of said Pax genes is over-expressed whereas the second Pax gene is under-expressed in said malignant state. Finally, surgical removal or chemotherapeutic treatment of pancreatic tumors in humans often leaves the patient without a significant number of insulin producing cells. Pax4 and optionally Pax6 may be used in tissue engineering (Langer and Vacanti, Science 260 (1993), 920-924) for the development of functional substitute for missing or damaged β-cells. Pancreatic tumor cells that have reverted in vitro to a normal levels of Pax4 expression, and optionally of Pax6 expression, can be re-introduced into the patient so that the said patient is provided with normal insulin producing cells of his own genetic background thereby reducing the risk of immunological rejection of the cells.

In a further preferred embodiment of the present invention, the testing for differentiation status in pancreatic cells is a testing for the developmental status in β-cells, which as shown by the examples, is indicative of juvenile diabetes. Juvenile diabetes is often the result of deficiency or failure in β-cell development. The examples show that deficiency in Pax4-expression is indicative of deficiency or failure in b-cell development and ergo insulin production (and thus diabetes such as juvenile diabetes).

Early diagnosis of juvenile diabetes is particularly advantageous and of considerable medical importance. Thus, it is a preferred embodiment to employ methods of the invention for diagnosis or detection of diabetes. This preferred embodiment can be used to diagnose juvenile diabetes in the coronar villi, i.e. prior to the implantation of the embryo. Furthermore, juvenile diabetes can, with the method of the present invention, be diagnosed via amniocentesis. The early diagnosis of juvenile diabetes in accordance with all applications of the method of the invention allows for treatment directly after birth before the onset of clinical symptoms.

In a particularly preferred embodiment of the method of the invention, said testing for differentiation status in said β-cells is carried out in an embryonic or newborn mammal.

As has been indicated hereinabove, it is particularly preferred to include at least one further step in the method of the invention, which is specific for different pharmaceutical and genetic therapeutic approaches. As mentioned above, different small pharmaceutically active molecules could be used to activate Pax4 and optionally Pax6 expression and therefore induce differentiation and production of insulin producing β-cells. Likewise, intracellular targeting of active Pax4 and/or optionally Pax6 would advance similar consequences. In accordance with this statement, pancreatic ductal epithelial cells have been proposed to contain potential stem cells for endocrine cell types. Induction of Pax4 activity in said cells but not exclusively in said cells can promote differentiation into insulin producing cells (Parsa et al, 1985, Cancer Res. 45:1285-1290).

In a further preferred embodiment of the method of the invention, said differentiation status of pancreatic cells is the result of the activity of a medicament or of a gene therapy approach. For example, said differentiation status may be influenced by gene therapy approaches where a functional Pax4 and optionally Pax6 gene is introduced *in vivo* into cells using a retroviral vector (Naldini et al., Science 272 (1996), 263-267; Mulligan, Science 260 (1993), 926-932) or another appropriate vector. Likewise, in accordance with the present invention cells from a patient can be isolated, modified in vitro to differentiate into β-cells using standard tissue culture techniques and reintroduced into the patient.

In a particularly preferred embodiment, said medicament or gene therapy approach affects the expression level of the Pax4 gene and, optionally, of the Pax6 gene at the mRNA or protein level.

The above embodiments of the present invention allow, *inter alia,* testing of a medicament for its influence on expression of the aforementioned Pax genes. As has been stated further hereinabove, abnormal expression levels of Pax4 and optionally of Pax6 are expected to be a causative agent in the generation of, for example, solid tumors of the pancreas. The method of the invention thus allows the testing of medicaments, the application of which would allow the cell to return to a normal expression level. Said normal expression level would be expected to have a direct influence on, e.g., the malignancy of a cell. For example, if a disease or tumor is a direct or indirect result of an under-expression of Pax4, the physician treating the respective patient would administer a medicament that stimulates expression of Pax4.

In a further preferred embodiment of the method the invention, the testing for differentiation status in pancreatic cells is a testing for the developmental status in Pax4 knockout mice that are optionally at the same time Pax6 knockout mice.

In an additional preferred embodiment of the method of the present invention, said method further comprises introducing a functional and expressible Pax4 gene and optionally further comprising introducing a functional and expressible Pax6 gene into pancreatic α-cells or ductal epithelial cells which possess a similar yet different differentiation pathway as compared to β-cells. With this embodiment of the invention, the person skilled in the art is in the position to redirect the fate of α-cells or ductal epithelial cells into β-cells. Thus, α-cells are expected to differentiate after transfection with the Pax4 gene and optionally the Pax6 gene into β-cells. A corresponding pharmaceutical application is-envisaged, if a patient suffers from a β-cell- and thus insulin deficiency. It is envisaged that this method is carried out in vitro or in vivo.

The present invention further relates to a non-human transgenic mammal comprising at least one inactivated Pax4 allele.

The non-human transgenic animal of the present invention can advantageously be.used for monitoring the development of different cells, for example, in the pancreas. However, the use of the non-human transgenic mammal is not confined to the study of pancreatic development. Since Pax4 is, in accordance with the present invention, now believed to be a master regulator for β-cells, its influence can also be studied in other cell types of the body.

Since the non-human transgenic animal of the invention which is preferably a transgenic mouse in the homozygous state has severe pancreatic disorders that, in the case of transgenic mice, lead to death within three days after birth, said transgenic animal can further be used for the investigation of diseases associated with developmental disorders, in particular in the pancreas. Since the transgenic mice are deficient in insulin producing cells and present clinical symptoms similar to human patients suffering from juvenile diabetes, said mice can serve has an animal model for therapeutic and pharmaceutical research against juvenile diabetes.

Preferably, the non-human transgenic mammal of the invention further comprises at least one inactivated Pax6 allele.

This embodiment allows the study of the interaction of Pax4 and Pax6 on the development of the mammal or certain tissues thereof, in particular, of the pancreas. All the applications that have been herein before discussed with regard to the Pax4 transgenic mammal also apply to the mammal that carries two transgenes. It might be also desirable to inactivate Pax4 gene expression and optionally Pax6 gene expresssion at a certain stage of development and/or life of the non-human transgenic animal. This can be achieved by using, e.g., tissue specific developmental and/or cell regulated and/or inducible promoters which drive the expression of, e.g., an antisense or ribozyme directed against the RNA transcript encoding the Pax4 protein and optionally to the Pax6 encoding RNA. A suitable inducible system is for example the tetracycline-regulated gene expression as described, e.g., by Gossen and Bujard (Proc. Natl. Acad. Sci. 89 USA (1992), 5547-5551) and Gossen et al. (Trends Biotech. 12 (1994), 58-62). In another preferred embodiment of the invention said transgenic mammal is human, a mouse or a rat.

In accordance with the present invention, the transgenic animal may be homozygous or heterozygous for either inactivated Pax4 or inactivated Pax6 or for both inactivated genes.

Moreover, the present invention relates to the use of at least one first nucleic acid sequence encoding a functional and expressible Pax4 protein and optionally a second nucleic acid sequence encoding a functional and expressible Pax6 protein for the preparation of a pharmaceutical composition for treating, preventing and/or delaying diabetes in a mammal. According to the invention, vectors containing said nucleic acid sequences may be operatively linked to regulatory elements allowing for expression of said nucleic acid sequences and/or for targeting of said nucleic acid sequences to pancreatic cells.

Further; the invention relates to the use of a functional Pax4 protein and optionally a functional Pax6 protein for the preparation of a pharmaceutical composition for the treatment, prevention and/or delay of diabetes in a mammal. The term "functional" bears the same meaning as outlined hereinabove.

Preferably, the mammal wherein diabetes is treated, prevented and/or delayed is a human, a rat or a mouse. The invention further comprehends a non-human transgenic mammal modified so as to comprise at least one first nucleic acid molecule comprising a sequence encoding a functional and expressible Pax4 protein and optionally a second nucleic acid molecule comprising a sequence encoding a functional and expressible Pax6 protein (wherein the mammal has expression of the nucleic acid molecule (s)). The mammal can be modified pre-natally or post-natally, e.g., after a method of the present invention shows low, impaired or no Pax4 protein or mRNA, for treatment, prevention or delaying diabetes; and, the modification can be by techniques discussed herein or by techniques within the ambit of the skilled artisan, without undue experimentation from this disclosure.

It is envisaged by the present invention that the nucleic acids and proteins are administered either alone or in any combination, and optionally together with a pharmaceutically acceptable carrier or excipient. Said nucleic acid sequences may be stably integrated into the genome of the mammal. On the other hand, viral vectors may be used which are specific for certain cells or tissues, preferably pancreas and/or brain, and which persist in said cells thereby conferring expression of the Pax genes in said cells. Suitable pharmaceutical carriers and excipients are well known in the art. Elements capable of targeting a nucleic acid molecule and/or protein to specific cells are described in the prior art, for example in, Somia et al., Proc. Natl. Acad. Sci. USA 92 (1995), 7570-7574. The pharmaceutical compositions can be administered to the mammal at a suitable dose, which can be determined from this disclosure and knowledge in the art, without undue experimentation by the skilled artisan taking into consideration typical factors such as the species, age, sex, weight, condition of the mammal, the route of administration, whether a Pax4 protein or Pax4 and Pax6 proteins are being administered, whether an agent for inhibiting or stimulating Pax4 or Pax4 and Pax6 is being administered, whether a nucleic acid or acids are being administered, and whether the nucleic acid or acids are for expression of Pax4 or Pax4 and Pax6 or are for inhibiting expression of Pax4 or Pax4 and Pax6, inter alia. A typical dose can be, for example, in the range of 0.001 to 1000 µg (or of nucleic acid for expression or for inhibition of expression in this range); however, doses below or above this exemplary range are envisioned, especially considering the aforementioned factors.

Administration of the suitable compositions may be effected in different ways, e.g., by intravenous, intraperitoneal, subcutaneous, intramuscular, topical or intradermal administration. The pharmaceutical compositions prepared according to the invention can be used for the prevention or treatment or delaying of different kinds of diseases, for example, pancreas related diseases, namely diabetes and related disorders. Said diseases and disorders are preferably derived from endocrine tissue, e.g. pancreas.

Furthermore, it is possible to use a pharmaceutical composition which comprises a nucleic acid sequence which encodes a Pax4 protein and optionally a nucleic acid sequence encoding a Pax6 protein for gene therapy. Naturally, both sequences may also be comprised in the same vector. As described above, the diseases often evolve when cells lose both functional copies of the Pax genes.- In such a case, introduction of functional copies of the corresponding gene may help to ameliorate the situation. For example, research pertaining to gene transfer into cells of the germ line is one of the fastest growing fields in reproductive biology. Gene therapy, which is based on introducing therapeutic genes into cells by ex-vivo or in-vivo techniques is one of the most important applications of gene transfer. Suitable vectors and methods for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO94/29469 or WO 97/00957, and references cited therein. The Pax4 and optionally Pax6 encoding gene(s) may be designed for direct introduction or for introduction via liposomes, or viral vectors (e.g. adenoviral, retroviral) into the cell. For example, the sequences encoding the human Pax4 and optionally the Pax6 gene may be operatively linked to regulatory sequences allowing the expression in the target cells and tissue. In genetic diseases the introduction of a normal or a functionally adequate allele of a mutated nuclear gene represents gene replacement therapy (see, e.g., Mouellic, Proc. Natl. Acad. Sci. USA, 87 (1990), 4712-4716; Joyner, Gene Targeting, A Practical Approach, Oxford University Press), which is applicable mainly to monogenetic recessive disorders such as, for example, diabetes and hypoglycemia.

Thus, in a further embodiment, the invention relates to a method for treating diabetes comprising:
(a) removal of a cell from a mammal;
(b) introduction of a functional and expressible Pax4 gene and optionally a functional and expressible Pax6 gene into said cell; and
(c) reintroducing the cell obtained as a product of step (b) into said mammal or into a mammal of the same species.

Yet, in a further embodiment, the invention relates to a method for treating a neuronal disorder comprising:
(a) removal of a cell from a mammal;
(b) introduction of a functional and expressible Pax4 gene and optionally a functional and expressible Pax6 gene into said cell; and
(c) reintroducing the cell obtained as a product of step (b) into said mammal or into a mammal of the same species.

It is to be understood that the introduced genes are functional and expressible after introduction into said cell and preferably remain in this status during the lifetime of said cell.

Preferably, said mammal is a human, a rat or a mouse.

In a preferred embodiment of the method of the invention said cell is a non-human germ line cell or non-human embryonic cell or derived therefrom. An embryonic cell can be for example an embryonic stem cell as described in, e.g., Nagy, Proc. Natl. Acad. Sci. 90 (1993) 8424-8428. In a further preferred embodiment, said cell is an non-human egg cell or derived therefrom.

Suitable vectors and methods for in-vitro or in-vivo gene therapy are described in the literature and are known to the person skilled in the art. The pharmaceutical compositions according to the invention can be used for the treatment of kinds of diseases hitherto unknown as being related to the expression and/or non-expression of the Pax4 gene and/or the Pax6 gene.

These and other embodiments are disclosed and encompassed by the description and examples of the present invention. For example, further literature concerning any one of the methods, uses and compounds to be employed in accordance with the present invention may be retrieved from public libraries, using for example electronic devices. For example the public database "Medline" may be utilized which is available on the Internet, for example under http://www.ncbi.nlm.nih.gov/PubMed/medline.html. Further databases and addresses, such as http://www-ncbi.nlm.nih.gov/, http://www.infobiogen.fr/, http://www.fmi.ch/biology/research-tools.html, http://www.tigr.org/, are known to the person skilled in the art and can also be obtained using, e.g., http://www.lycos.com. An overview of patent information in biotechnology and a survey of relevant sources of patent information useful for retrospective searching and for current awareness is given in Berks, TIBTECH 12 (1994), 352-364.

### BRIEF DESCRIPTION OF THE DRAWINGS

This disclosure may best be understood in conjunction with the accompanying drawings, incorporated herein by reference, which show:

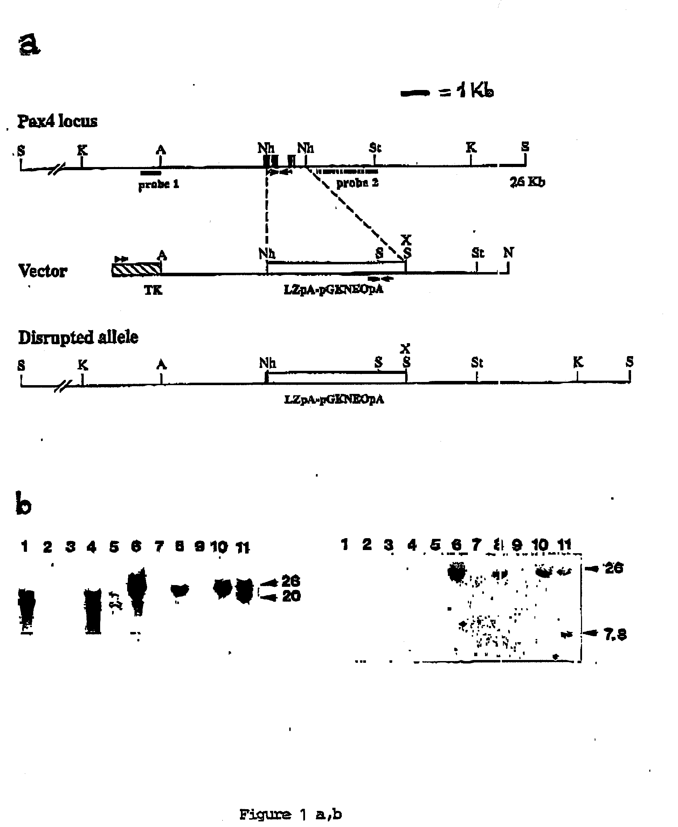

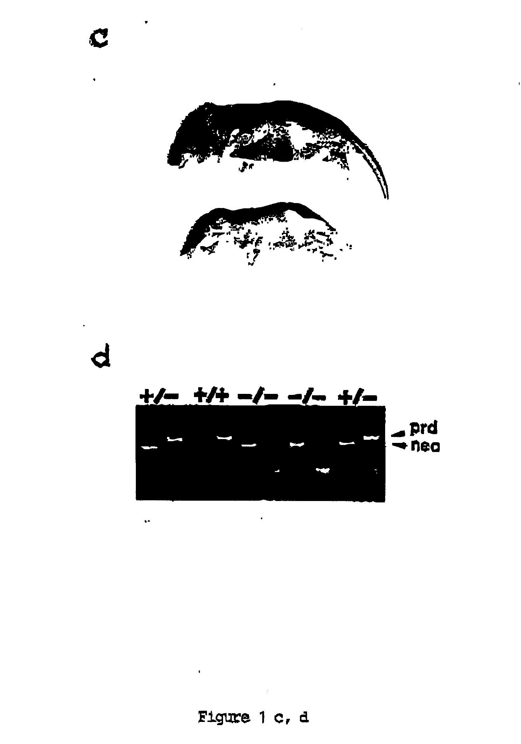

Figure 1 (Figs. 1a, 1b, 1c, 1d). Targeted disruption of Pax4 and generation of Pax4 (-/-) mice
   a, structure of the wild-type and targeted Pax4 loci. A targeted deletion of almost all the entire paired box domain (dark boxes; exons 2, 3 and 4) of Pax4 was produced, by fusing in frame a β-galactosidase-neomycin resistance cassette (transcription direction is indicated by double arrowheads). Restriction enzymes: A, ApaI; K, KpnI; Nh, NheI; N, NotI; S, SpeI; St, StuI; X, XhoI.
   b, DNA isolated from ES cell clones was digested with SpeI and analyzed by Southern blot hybridization with both, a 0.7 kb external genomic fragment (probe 1, left), and a 0.5 kb cDNA fragment (probe 2, right). Sizes are in kilobases. In both, the 26 kb fragment is indicative of the wild-type allele, while the 20 kb and the 7.8 kb fragments, respectively, originate from the targeted allele. Asterisks indicate two clones positive for homologous recombination.
   c, A pair of 2 days-old littermates, wild-type (upper) and Pax4-deficient (lower), showing the reduced size of the null-mutant mouse.
   d, Embryos were genotyped by PCR analysis using genomic DNA from yolk sacs or tails, and two sets of primers (located as shown by arrows in a), in order to amplify wildtype-paired domain and/or, the neo gene.

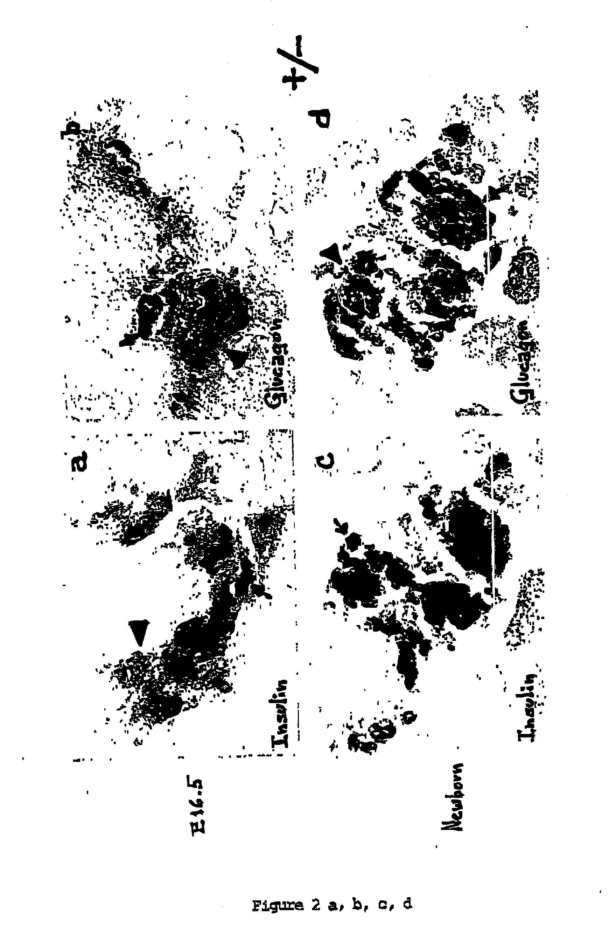

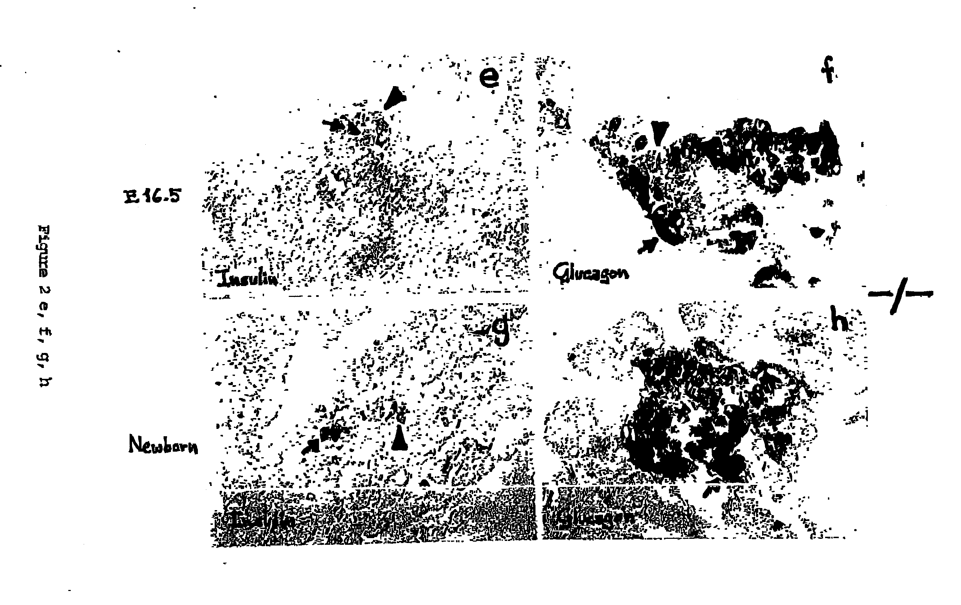

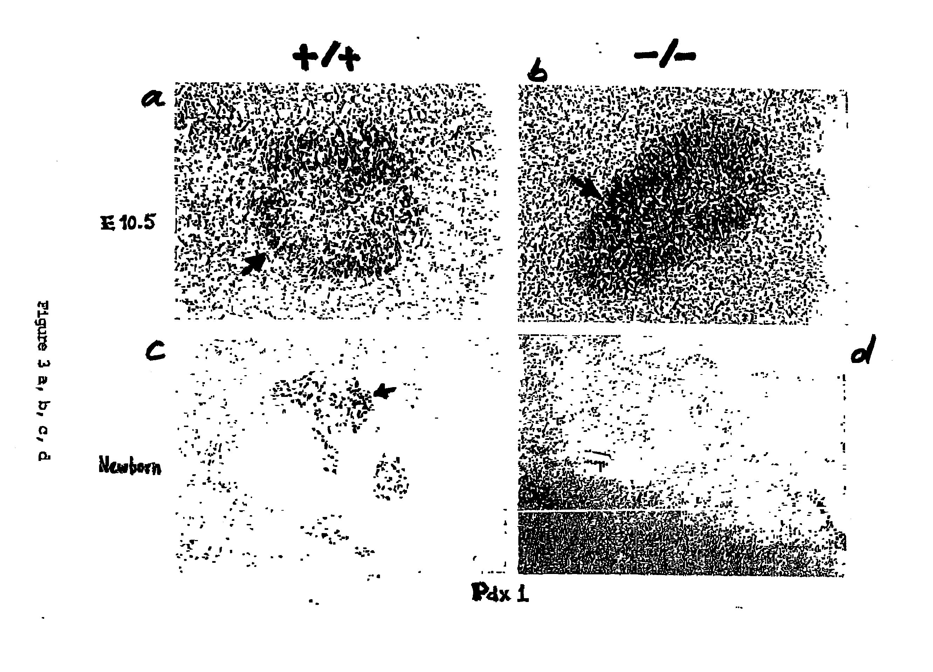

Figure 2 (Figs. 2a, 2b, 2c, 2d, 2e, 2f, 2g, 2h). Analysis of LacZ activity, insulin and glucagon expression, in the pancreas of Pax4 (+/-) and Pax4 (-/-) E16.5 embryos and newborn mice
   In E16.5 (+/-) pancreas, insulin (arrow in a) and glucagon (arrow in b) cells are associated with regions where LacZ activity (arrowheads in a and b) is detected. In contrast, in the pancreas of E16.5 (-/-) embryos, expression of LacZ (arrowheads in e) is diminished, whereas insulin is absent (compare a and e).
   In E16.5 (-/-) pancreas a larger number of glucagon-cells is found (arrow in f), associated with regions of LacZ activity (arrowhead in f). In (+/-) newborn pancreas, LacZ expression is restricted to insulin cells (arrow in c), and appears surrounded by the glucagon-cells (arrow in d). In (-/-) newborn pancreas, no expression of LacZ activity or insulin is detected (compare c, d and g). Meanwhile, in (-/-) newborn pancreas, numerous glucagon-cells are present (arrow in h), also abnormally distributed in clusters (compare d and h). Magnification is 400X.

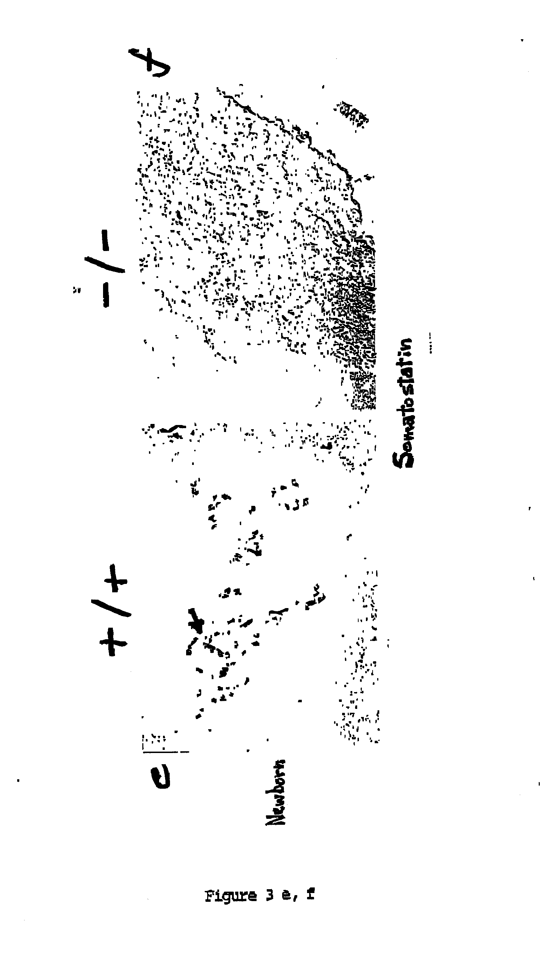

Figure 3 (Figs. 3a, 3b, 3c, 3d, 3e, 3f). Expression of Pdx1 and somatostatin in Pax4 (+/+) and (-/-) newborn pancreas
   In the dorsal pancreas of both, (+/+) and (-/-) E10.5 embryos, the early expression of Pdx1 looks similar (compare a and b).
   In newborn (+/+) pancreas, Pdx1 is restricted to mature insulin-producing β-cells (arrows in c). In the pancreas of Pax4 (-/-) newborn mice, no expression of Pdx1 is detected (compare c and d). In (+/+) newborn pancreas, somatostatin producing δ-cells are mainly distributed within the cells that surround the Islets of Langerhans (arrows in e). In (-/-) newborn pancreas, however, somatostatin is not detected (compare e and f). a and b are vibratome sections. c and d are cryostat sections. e and f are paraffin sections. Magnification in (c-f) is (200X), and in a and b is (400X).

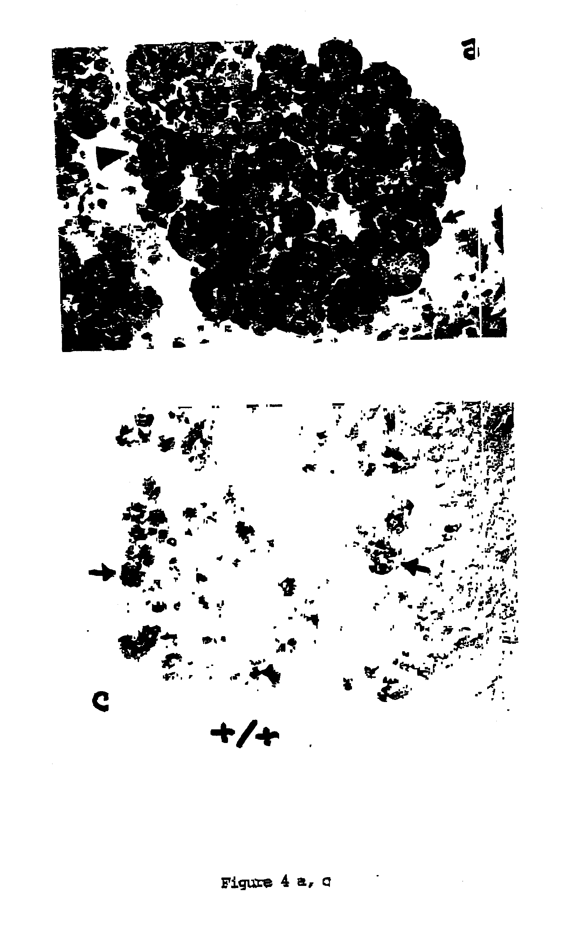

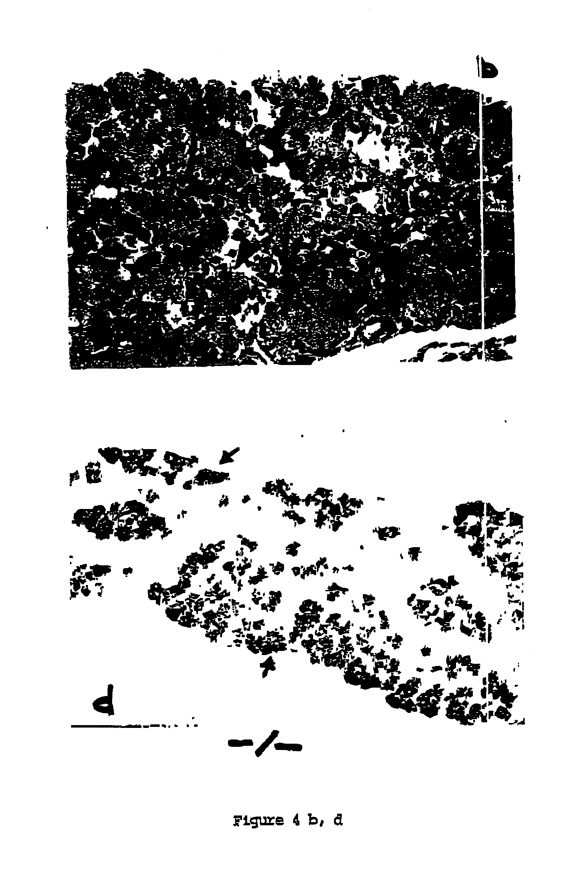

Figure 4 (Figs. 4a, 4b, 4c, 4d). Histological analysis of Pax4 (+/+) and (-/-) pancreas from newborn mice, and expression of α-amylase
   Exocrine acini are shown in (a), containing groups of pyramidally-shaped cells (arrowheads), with their nuclei at the base. Exocrine cells are also present in newborn (-/-) pancreas (arrowheads). However, extensive areas of cytoplasm are observed, as well as a disorganized distribution of their nuclei (compare a and b). In the (+/+) newborn pancreas, exocrine cells contain different amounts of α-amylase (arrows and arrowhead in ac), reflecting the depletion of digestive enzymes that normally follows suckling. In almost all the exocrine tissue of newborn (-/-) pancreas, however, a large amount of α-amylase is present (arrows in e), indicating that its secretion might be affected. Magnification in a and b is (400X), and in c and d is (200X).

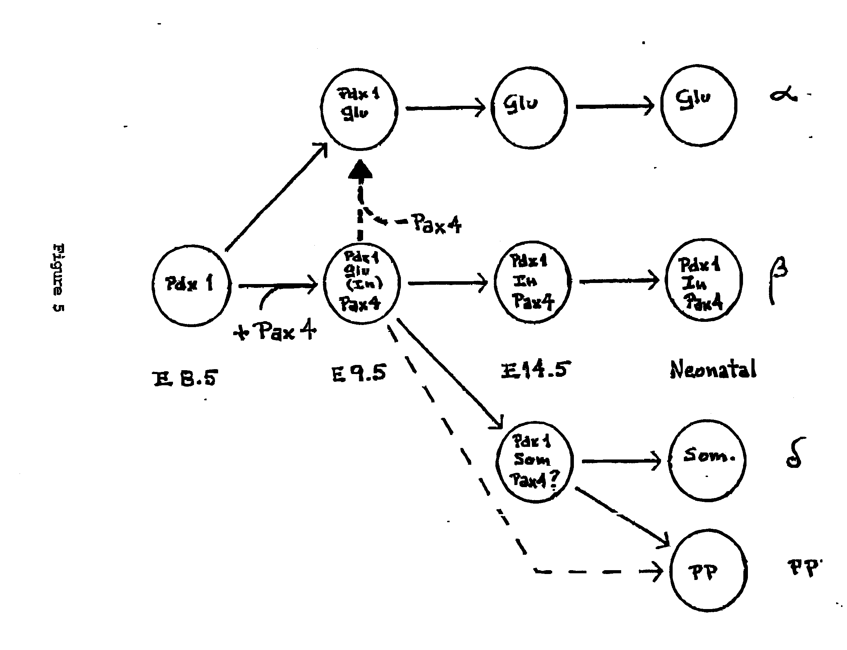

Figure 5. Model for the role of Pax4 in endocrine differentiation, in the mouse pancreas.
   Earliest endocrine progenitor cells are characterized by their expression of Pdx1, at around E8.5 of mouse development (Guz et al., Development 121 (1995), 11-18). Differentiation of α-cells occurs very early in development, in precursors in which presumably Pdx1 is no longer present. In the earliest β-cell progenitors, genes like Pdx1 and insulin are selectively expressed, and maintenance of their expression seems important for their progression into differentiation. In mature endocrine cells: Pdx1, insulin and Pax4 have their expression restricted to the β-lineage. Pax4 may promote and/or maintain the expression of genes specific for the insulin-producing β-cells.
Figure 6. Hormonal concentrations in pancreata of wild type, heterozygous and homozygous Pax4 mutant newborn mice
   Little or no differences in insulin or glucagon concentration was observed between wild type and heterozygous animals. Homozygous animals showed almost complete reduction in insulin concentration and a 3 fold increase in glucagon levels.
Figure 7. Northern blot analysis of pancreatic tumor cell lines

Cell line AR42J contained two Pax4 RNA transcripts: one that is similar in size to the wild type transcript (arrowhead) and a second that is larger in size (star). Weak Pax4 expression is detected in cell lines βTC-1 and αTC 1-9.

A better understanding of the present invention and of its many advantages will be had from the following examples, given by way of illustration.

### EXAMPLES

Antibodies used against insulin, glucagon and amylase are commercially available from several companies. The antibodies used in Applicants' experiments were purchased from Boeringer Mannheim. The Pdx1 antibody was a generous gift from Prof. Thomas Edlund (Department of Microbiology, University of Umea, S-901 87 Umea, Sweden). Antibodies with the same specificity i.e. which specifically recognize the Pdx1 protein can be prepared using the Pdx1 protein as an antigen according to conventional procedures.

### Example 1: Preparation of construct for homologous recombination and generation of Pax4-deficient mice

Targeted deletion of almost all the entire paired box domain (dark boxes; exons 2, 3 and 4) of Pax4 was produced, by fusing in frame a β-galactosidase-neomycin resistance cassette. A 5.1 kb Xba-Xhol fragment containing LacZpA-pGKNeopA sequences was blunt ended and ligated into the Nhel-digested and blunt ended Pax4 construct. The LacZpA-pGKNeopA construct was made by combining the pGKneo plasmid (Soriano et al., 1991, Cell 64:693-702) and the β-galactosidase gene which is available from several commercial sources (e.g. pCH110 vector from Pharmacia) .
The 5' Nhel site was retained and a new Spel site was generated at the 3' end. A F9 polyoma early promoter derived HSV TK gene was added upstream to the 5' homology, for negative selection. R1 ES-cells were electroporated and selected following standard procedures. Positive clones were used to generate chimeras by morula aggregation. Tail and yolk sac DNA-isolation and PCR amplification of genomic DNA were performed using standard procedures. Analysis of the phenotype was done in NMR1 and C57BL/6 mice. No differences were observed between both genetic backgrounds.

### Example 2: Studies on the Expression of Pax4 during Development

A genomic screen has previously identified Pax4 as a member of the murine family of Pax genes (Walther et al., Genomics 11 (1991), 424-434). By RT-PCR Applicants isolated the corresponding cDNA sequence.

PCR amplification of a fragment (615 nt) encoding the paired and homeobox domains of Pax4 cDNA was done with 1 µg of total RNA isolated from E13.5 mouse embryos. Reverse transcription was performed with a kit from Pharmacia, and random primers. Primers used were obtained after sequencing the corresponding genomic regions encoding the paired (5', sense primer: 5' AGC AAT AAG AGG GAT GCG ACC 3' (SEQ ID No. 5)) and homeobox (3', antisense: 5' AGC TGT GCT TCC CAT TTC AGC 3' (SEQ ID No. 6)) domains, respectively. In all the PCR reactions that were performed (including 5' and 3' RACE), Taq polymerase was added after the first denaturation step (95°C, 3 min), before cycling proceeded. 2 units of Taq polymerase together with 1 unit of "Perfect Match" (from Stratagene) were added. 35 cycles were performed: 95°C, 1½ min; 60°C 1½ min; 72°C 2½ min. A final step with: 60°C, 1½ min and 72°C for 10 min was performed. A faint band with the expected size was seen after gel electrophoresis in 2% low-melting agarose. This was excised and reamplified (5 µl of molten agarose) with the same program. DNA was electroeluted from a 2% preparative gel, filled-in and blunt-end cloned into Bluescript digested with SmaI, following standard procedures. Sequencing of the isolated clones was done with a sequencing kit from Pharmacia. 5'-RACE amplification was performed using a kit from GIBCO-BRL, following manufacturer's instructions. Briefly, 5 µg of total RNA isolated from the posterior region of E10.5 mouse embryos (posterior to the hindlimb bud: "tail-RNA") was used as template for first- strand cDNA synthesis. Two sets of nested primers were used in combination with the AP (primer BS41: 5' GCG AAT TCC CTG AAG TGC CCG AAG TAC TCG ATT 3' (SEQ ID No. 7)) and UAP (primer BS57: 5' GGC TCC GTG AAA TGT CAC AG 3' (SEQ ID No. 8)) primers, respectively. In the first PCR reaction, one primer specific for Pax4 located close to the 5' region of its known sequence was used in conjunction with the "AP" primer 5' CGT AGT ACT GTC GAC TAG CAG GGI IGG GII GGG IIG 3' (SEQ ID No. 9) from the commercially available kit (5' Race system from GIBCO-BRL). The "AP" primer contains an adaptor linked to a dG-tail which anneals to the dC-tailed cDNA. For the second reaction, a "nested" Pax4 primer ("nested meaning located more 5' or 'upstream' to the Pax4 primer used in the first reaction) was used together with the commercial "UAP" primer (primer that contains only the adaptor sequence of the AP primer 5' CGT AGT ACT GTC GAC TAG CA 3' (SEQ ID No. 10). First PCR (AP/BS41 primers) was performed with 5 µl of dC-tailed cDNA (35 cycles of: 94°C, 1½ min; 55°C, 1½ min; 72°C, 3 min). 2 µl of this resulting PCR reaction (diluted 20 times) were reamplified with primers UAP/BS57, and 40 cycles of: 94°C, 1 min; 60°C, 1 min; 72°C, 2½ min. A single band of approximately 500 nt was seen after gel electrophoresis.
This was electroeluted and cloned into the TA-pGEM vector (PROMEGA). 180 nt of 5' new sequence information was obtained with this approach. 3' RACE amplification was done using 2.5 µg of "tail" E10.5 RNA as template. First-strand cDNA synthesis was performed with the NotI-oligo dT primer provided by the First-strand cDNA synthesis kit (Pharmacia). First PCR was performed with the NotI primer (5' AAC TGG AAG AAT TCG CGG CCG CAG GAA 3' (SEQ ID No. 11)) and BS36 (5' CAG GAA GAC CAG AGC TTG CAC TGG 3' (SEQ ID No. 12)) primers. 35 cycles (94°C, 1½ min; 55°C, 1½ min; 72°C, 3 min) were performed. 2 µl of this PCR reaction (diluted 20 times) were reamplified with a nested primer (BS42: 5' GCG GAT CCC ACA GGA ATC GGG CTA TCT TC 3' (SEQ ID No. 13)) and NotI primer. After gel electrophoresis, a prominent band of approximately 550 nt was excised from a 2% low-melting agarose gel. 2 µl of molten agarose were reamplified with another nested primer (BS59: 5' GCG CAG GCA AGA GAA GCT GA 3' (SEQ ID No. 14)) and NotI primer. The last two PCRs were: 40 cycles of: 60°C, 1½ min; 72°C, 3 min; 94°C, 1½ min. A 420 nt prominent band was electroeluted and subcloned into TA-pGEM vector. The fidelity of the amplified 5' and 3'-PCR fragments was assessed by two procedures: first, a 1.1 kb fragment was amplified from "tail" E10.5 RNA, using primers (sense primer: 5' CTT CCA GAA GGA GCT CTC 3' (SEQ ID No. 15) and its antisense primer: 5' TGG GAT GAT GGC ACT TGT C 3' (SEQ ID No. 16)) designed from the most 5' and 3' newly sequences. Its size and sequence were as expected. Also, the sequence of the three PCR-isolated fragments (5'-, 615 nt and 3'-) was compared with the equivalent coding regions in a genomic clone.

Analysis of Pax4 expression revealed that its transcripts are restricted to a few cells in the ventral spinal cord and the early developing pancreas. In order to investigate the function of this gene during development, Applicants generated Pax4-deficient mice; see Example 1. Inactivation of Pax4 after homologous recombination in ES-cells was achieved, after deleting almost the entire paired box domain, and fusing the β-galactosidase gene in frame with the amino-terminus of Pax4 (Figure 1a). This approach also allowed Applicants to analyze in more detail the expression of Pax4 throughout development, by detection of LacZ activity.

Heterozygous mice do not exhibit any obvious abnormalities and they are viable and fertile. Staining of (+/-) embryos at day 10.5 of development (E10.5), revealed LacZ activity in cells within the dorsal pancreas. For detection of LacZ activity, X-gal staining of mouse embryos and isolated newborn pancreas was performed, following standard procedures. A post-fixation was performed in 4% paraformaldehyde, at 4°C, overnight. For immunohistochemistry, X-gal stained embryos and tissues were embedded in paraffin, and sectioned (10 µM) with a microtome. Expression of LacZ in the pancreas of Pax4 (+/-) embryos proceeded until birth. In heterozygous newborn pancreas, LacZ activity was found restricted to discrete areas corresponding to the β-cells in the Islets of Langerhans, as judged by co-expression of LacZ and insulin (Figures 1c and d).

Pax4-deficient offspring were born with the expected Mendelian distribution, indicating that the absence of Pax4 is not lethal in utero. At birth, Pax4 (-/-) mice appear normal and were indistinguishable from their littermates. However, 48 hours later they exhibited growth retardation and dehydration (Figure 1c). All Pax4-deficient mice died within the first three days after birth, demonstrating the complete penetrance of the mutant phenotype. Pancreas of newborn Pax4 (-/-) mice showed a normal macroscopical appearance. The expression of LacZ in the pancreas of Pax4 (-/-) mutant mice was also investigated. In E10.5 (-/-) embryos LacZ activity was detected in cells of the dorsal pancreas, in a similar manner to heterozygous embryos. No differences in the expression of LacZ activity were observed in the developing pancreas of Pax4 (+/-) and (-/-) embryos, until approximately E16.5. After this, however, in the pancreas of Pax4-deficient mice LacZ expression began to diminish (compare Figures 2a, b and 2e, f), and it became undetectable after birth (compare Figures 2d and 2g).

### Example 3: Expression of Insulin and Glucagon in Pax4 Targeted Mice

In the pancreas, all endocrine cells arise from common multipotent precursors (Alpert, Cell 53 (1988), 295-308). The first precursor cells containing insulin and glucagon, appear around E9.5 (Gittes et al., Proc. Natl. Acad. Sci. USA 89 (1992), 1128-1132; Teitelman et al., Development 118 (1993), 1031-1039). In the mouse pancreas, differentiation of exocrine and most endocrine cells starts around E16.5 of development (Githens, The Pancreas: biology, pathobiology, and disease. Second Edition (ed. Vay Liang W. Go, et al.) Raven, New York, (1993), 21-73). At birth, insulin production is mostly restricted to fully differentiated β-cells, located in the center of the islets of Langerhans. These are surrounded by the glucagon-producing α-cells. β-cells comprise the majority of the endocrine population, whereas the α-cells represent only a small fraction (Slack, Development 121 (1995), 1569-1580). Applicants have tested for the expression of insulin and glucagon in the pancreas of Pax4 heterozygous and Pax4 null-mutant mice, by immunochemistry on paraffin sections performed as previously described (Oliver et al., EMBO J. 7 (1988), 3199-3209).

At E10.5, insulin-producing cells were detected in the pancreas of Pax4 (+/-) and (-/-) embryos. In E16.5 heterozygous embryos, all insulin cells were found included within the area of LacZ activity (Figure 2a). As previously mentioned, in heterozygous newborn mice LacZ and insulin were detected in the same cells (Figure 2c), indicating that later in development Pax4 expression is restricted to the insulin-producing β-cells. In contrast, in the pancreas of Pax4 (-/-) E16.5 embryos and newborn mice, few if any insulin-producing cells were detected (Figure 2e, g). This indicates that in the absence of Pax4, the maturation of the pancreatic β-cells was affected. In the pancreas of E10.5 (+/-) and (-/-) embryos, cells containing glucagon are present. In heterozygous E16.5 embryos, glucagon was detected in cells, most of which were contained within the area of LacZ activity (Figure 2b). In newborn heterozygous pancreas, glucagon was present in cells surrounding the area where the LacZ was expressed, but were not included within it (Figure 2d). In the pancreas of Pax4 (-/-) E16.5 embryos and newborn mice, a considerably larger number of glucagon-producing cells was found. These cells also showed an aberrantly clustered distribution (Figures 2f and h).

### Example 4: Expression of Pdx1 in Pax4 Targeted Mice

In order to further confirm that in the pancreas of Pax4-deficient newborn mice β-cells are missing, the expression of a specific β-cell marker, Pdx1, was tested. This gene appears very early in pancreas development (most likely in the earliest pancreatic progenitors), but later becomes restricted to the fully differentiated β-cells (Guz et al., Development 121, 11-18 (1995); Miller et al., EMBO J. 13 (1994), 1145-1156) (Figure 3c). Its early expression was assayed by whole-mount immunostaining of E10.5 mouse embryos, using an established protocol (Ohlsson et al., EMBO J. 13 (1994), 1145-1158), except that bleaching was for 24 hours. After staining, E10.5 embryos were postfixed, embedded in a gelatin-BSA matrix and sectioned with a vibratome. Expression of Pdx1 in newborn pancreas by immunostaining on cryostat sections was performed as previously described, with the following modifications: newborn pancreas was fixed 3 hours with 4% paraformaldehyde, and then cryoprotected with 30% sucrose in PBS, overnight. After washing-off the secondary antibody, secretions were incubated with 0.6% H₂O₂, 20 min. At E10.5, Pdx1 was similarly expressed in the pancreas of both, Pax4 (+/+) and (-/-) embryos (Figures 3a,b). However, in the pancreas of Pax4-deficient newborn mice, Pdx1 expression was not detected. This confirms the initial conclusion that, in the pancreas of Pax4 null-mutant mice, the mature β-cells are absent. It also suggests that, although Pax4 may not be required for generation of the earliest endocrine precursors, it is crucial for β-cell differentiation.

### Example 5: Expression of Somatostatin in Pax4 Targeted Mice

During mouse embryogenesis, somatostatin-producing cells start to differentiate later than α- and β-cells (Teitelman et al., Development 118 (1993), 1031-1039). In the pancreas of Pax4 (+/+) newborn nice, somatostatin-producing δ-cells were mainly distributed in the periphery of islets, intermingled with the α-cells (Figure 3e), suggesting that inactivation of Pax4 also affected the maturation of the 8-lineage. Expression of somatostatin in the gut, however, appeared unaffected.

### Example 6: Histological Analysis of Newborn Pancreas From Pax4 Targeted Mice

Exocrine pancreas is a lobulated, branched, acinar gland, with pyramidal-shaped secretory cells and basal nuclei (Pictet, Devl. Biol. 29 (1972), 436-436) (Figure 4a). Histological analysis of newborn Pax4 (-/-) pancreas showed that in this gland, exocrine tissue was present. However, the cytoplasm of exocrine cells seemed expanded, and their nuclei did not show a homogenously basal distribution (Figure 4b). When pancreas of 3-days old wildtype mice was analyzed for the presence of a specific exocrine enzyme (α-amylase), large portions of exocrine tissue showed little or no expression of such marker (Figure 4c). This most likely reflects the depletion of exocrine digestive enzymes that normally occurs after suckling (Githens, The Pancreas: biology, pathobiology, and disease. Second Edition (ed. Vay Liang W. Go, et al.) Raven, New York, (1993), 21-73). At birth, Pax4-deficient mice are able to suckle, since their stomachs are full with milk (Figure 1c). However, 3-days old Pax4 (-/-) pancreas showed a strong expression of α-amylase in all exocrine cells, indicating that they might not be able to secrete their enzymes into the digestive tract (Figure 4d).

The conclusions to be drawn from the results of the above examples with respect to the influence of the Pax4 and Pdx1 genes on the development of pancreatic cells is shown in Figure 5.

### Example 7: Insulin and glucagon concentrations in pancreata of heterozygous and homozygous Pax4 mutant newborn mice

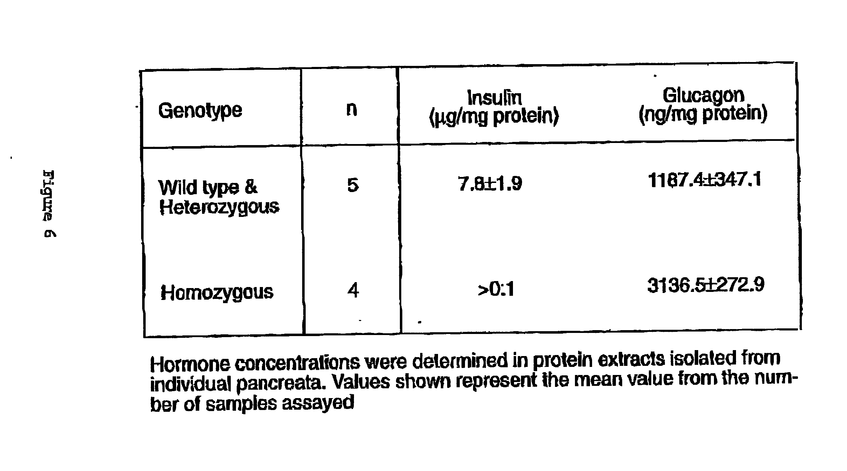

Insulin and glucagon production in complete pancreata from heterozygous or homozygous newborn Pax4-LacZ mice was quantified by radioimmunoassay (RIA). No significant differences in insulin or glucagon concentrations were observed between wild type and heterozygous animal (Figure 6). However, a severe and near total reduction in insulin concentrations was observed in homozygous animals while a three fold increase in glucagon levels was registered. These results are in agreement with those obtained by immunochemistry (example 3) which demonstrated a near complete absence of β-cells and a larger than normal α-cells population in the pancreata of mutant animals lacking Pax4. These observations demonstrate that Pax4 is required for β-cells generation during pancreatic development.
RIAs for insulin were performed using a commercially available kit from Linco Research (Cat#: RI-13K) and done according to the manufacturer's instructions. Pancreata from wild type and mutant mice were isolated just before birth by cesarean section at e19 of gestation. Individual pancreata were placed in 40 µl of acid extraction buffer (80 ml ethanol, 17.9 ml distilled water, 2.1 ml concentrated HCl) and sonicated on ice to extract their protein contents. After an overnight incubation at 4°C, samples were centrifuged 15 minutes at 13 000 rpm. Supernatants were then transferred into a new tube and the RIA were performed with a 1:1000 to 1:10 000 dilution of each protein extraction. Briefly, 100 µl of each diluted extract were incubated 18 hours at 4°C with 100 µl rat insulin antibody and 100 µl 125I-insulin label. Samples were then precipitated in 1 ml precipitating reagent for 20 minutes at 4°C and centrifuged 15 minutes at 2000 rpm. Supernatants were decanted and pellets counted in a scintillation counter. RIA for glucagon were performed using a commercially available kit from Linco Research (Cat#: GL-32K) according to the manufacturer's instructions. Proteins from newborn wild type and mutant pancreata were isolated as describe above and 100 µl of each diluted samples were incubated at 4°C for 18 hours with 100 µl rat glucagon antibody followed by 24 hours incubation at 4°C with 100 µl 125I-glucagon label. Samples were then precipitated in 1 ml precipitating reagent for 20 minutes at 4°C and centrifuged 15 minutes at 2000 rpm. Supernatants were decanted and pellets counted in a scintillation counter.

### Example 8: Expression of Pax4 in pancreatic tumor cell lines

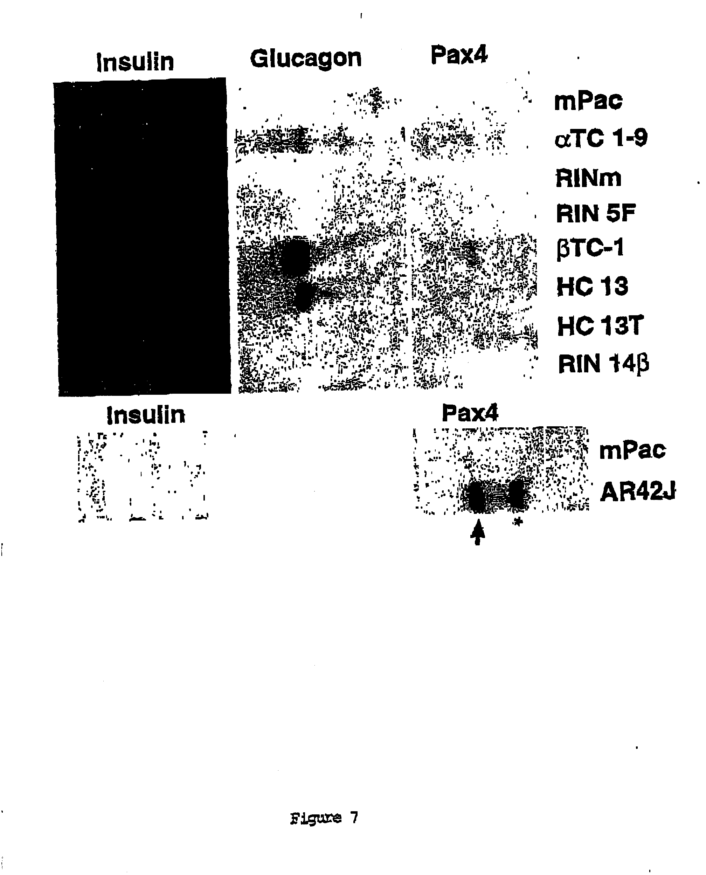

Pax4 expression levels in various insulinoma (RIN 5F, βTC1), glucagonoma (αTC 1-9), somatostatinoma (RIN 14β), islet tumor (HC 13, HC 13T, RINm), ductal adenocarcinoma (mPAC) and tumoral AR42J cell lines were analysed (Figure 7). Low levels of Pax4 expression was detected in βTC1 and αTC 1-9 cell lines which reflected endogenous low levels expression in β-cells of normal mice. No expression was observed in RINm, RIN 5F, RIN 14β, HC 13T and mPac lines.
Interestingly, high levels of Pax4 expression was detected in AR42J cells which are derived from a tumor in the exocrine tissue of the pancreas (reviewed in Christophe J, Am. J. Physiol. 266 (1994), G963-G971). Furthermore, a second larger RNA messenger transcript is detected in this cell line suggesting possible genomic modifications in one of the alleles of Pax4. Chromosomal rearrangements involving Pax genes have been associated with the development of certain tumors such as alveolar rhabdomyosarcomas (Galili et al., Nature Genetics 5 (1993), 230-205; Davis et al., Cancer Res. 54 (1994), 2869-2872). Results obtained in AR42J cells suggest that such a chromosomal rearrangements may occur within the Pax4 locus altering the properties of the protein and may be relevant to the tumorous phenotype.
Expression levels in the different cells lines were evaluated by conventional Northern blot analysis as describe, e.g., Sambrook et al., " Molecular Cloning: A Laboratory Manual", CSH Press, Cold Spring Harbor, 1989. Briefly, cells were trypsinized and transferred in a tube containing 1 ml homogenization buffer (3M LiCl, 6M Urea, 0.1% SDS) and sonicated 3-4 times 20 seconds on ice.
After overnight incubation at 4°C, lysates were centrifuged 30 minutes at 18600 rpm. RNA pellets were washed twice in 1 ml 3M LiCl, dissolved in 500 µl solubilization buffer (10mM Tris-Cl pH 7.5, 0.5% SDS) and purified by phenol-chloroform extraction followed by ethanol precipitation. 20 µg of RNA was migrated on an agarose gel and transferred onto a nylon membrane according to standard Northern blot protocols. Membranes were then hybridized with either a mouse Pax4 cDNA probe (EMBL accession # Y09584), an mouse glucagon cDNA probe (EMBL acc.# Z46845) or a rat insulin cDNA probe (EMBL acc.# J04807). Cell lines RINm (cat# CRL-2057), RIM 5F (cat# CRL-2058) and RIN 14β (cat# CRL-2059) were obtained from American Type Culture Collection. Cell lines βTC1, αTC 1-9, HC 13, HC 13T and mPac can be isolated from Rip1Tag2 transgenic mice (Hanahan, D., Nature 315 (1985),115-122) using conventional tissue culture techniques. Cell line AR42J can be derived from azaserine-induced malignant nodules from the rat pancreas (Longnecker et al., Cancer lett. 7 (1979), 197-202).

### Example 9: Cloning of additional Pax4 cDNA sequence

An additional Pax4 cDNA sequence was isolated from β-cell cDNA library. This sequence (SEQ ID No. 17) is identical to the nucleotide sequence as depicted in SEQ ID No. 1 with the exception of an additional G nucleotide after position 1131 which results in the usage of an alternative translational STOP codon at position 1214.
The resulting 11 amino acids in the C-terminal end of the protein generated by SEQ ID No. 17 are therefore modified and elongated by 17 amino acids when compared to the protein generated by SEQ ID No. 1.
The β-cell cDNA library can be prepared from the βTC1 cell line using a ZAP-cDNA synthesis kit from Stratagene (Cat# 200400) according to the manufacturer's instructions. Approximately 1 x 10⁶ clones of this library was plated, transferred to a nylon membrane and hybridized under high-stringency with a ³²P-labeled Pax4 probe (EMBL acc# Y09584). Hybridization was done overnight a 65°C in 6X SSPE, 5X Denhardts, 0.5% SDS, 0.1 mg/ml denatured salmon sperm DNA and followed by two 30 minutes wash at 65°C in 2XSSC/0.5% SDS and one 30 minutes wash at 65°C in 0.1XSSC/0.5% SDS. Positive clones were plaque purified and the resulting cDNA sequences were isolated using a rapid excision kit from Stratagene (Cat#211204). Nucleotide sequences were obtained using standard sequencing techniques as describe, e.g., Sambrook et al., " Molecular Cloning: A Laboratory Manual", CSH Press, Cold Spring Harbor, 1989.

## Claims

1. An in vitro method for testing the differentiation status in pancreatic cells of a mammal comprising
(a) determining the level or status of Pax4 mRNA in pancreatic cells of said mammal; and/or
(b) determining the level or status of Pax4 protein in pancreatic cells of said mammal; and
(c) comparing said level or status of Pax4 mRNA and/or Pax4 protein with the corresponding level in normal pancreatic cells,
wherein said determination and comparison of the status of Pax4 mRNA and/or Pax4 protein denotes the determination and comparison whether said mRNA or protein bears a mutation, deletion, or a post-translational modification which would affect the overall activity of the gene when compared to the wild-type normal gene product.

2. The method according to claim 1 further comprising
(d) determining the level or status of Pax6 mRNA in pancreatic cells of said mammal; and/or
(e) determining the level or status of Pax6 protein in pancreatic cells of said mammal; and
(f) comparing said level or status of Pax6 mRNA and/or Pax6 protein with the corresponding level in normal pancreatic cells,
wherein said determination and comparison of the status of Pax6 mRNA and/or Pax6 protein denotes the determination and comparison whether said mRNA or protein bears a mutation, deletion, or a post-translational modification which would affect the overall activity of the gene when compared to the wild-type normal gene product.

3. The method of claim 1 or 2, wherein said mammal is in the
(i) embryonic;
(ii) newborn; or
(iii) adult stage.

4. The method according to any one of claims 1 to 3. wherein said mammal is a mouse or wherein said mammal is a human.

5. The method according to any one of claims 1 to 4, wherein said determination in step (a) and optionally in step (d) is effected by employing
(i) a nucleic acid sequence corresponding to at least a part of the Pax4 gene and preferably encoding at least part of the Pax4 protein and optionally a second nucleic acid sequence corresponding to at least a part of the Pax6 gene and preferably encoding at least part of the Pax6 protein;
(ii) a nucleic acid sequence complementary to the nucleic acid sequence(s) of (i); or
(iii) a primer or a primer pair hybridizing to the nucleic acid sequence(s) of (i) or (ii).

6. The method according to any one of claims 1 to 4, wherein said determination in step (b) and optionally of step (e) is effected by employing an antibody or a fragment thereof that specifically binds to the Pax4 protein and optionally by employing a second antibody or a fragment thereof which specifically binds to the Pax6 protein.

7. The method according to any one of claims 1 to 6, wherein said pancreatic cells are β-cells or δ-cells.

8. The method according to any one of claims 1 to 7, wherein said developmental status is indicative of a malignancy or malignant potential of said pancreatic cell.

9. The method according to any one of claims 1 to 7, wherein said differentiation status in said β-cells is indicative of juvenile diabetes.

10. The method according to claim 9, wherein said differentiation status in said β-cells is carried out in the embryonic status or in the newbom status.

11. The method according to any one of claims 1 to 10, wherein said differentiation status is the result of the activity of a medicament or of a gene therapy approach.

12. The method according to claim 11, wherein said medicament affects the expression level of the Pax4 gene and optionally of the Pax6 gene at the mRNA or the protein level.

13. The method according to any one of claims 1 to 10, wherein said differentiation status is tested in Pax4 knockout mice that are, optionally, at the same time Pax6 knockout mice.

14. The method according to any one of claims 1 to 10, further comprising introducing the functional and expressible Pax4 gene and optionally further comprising introducing a functional and expressible Pax6 gene into pancreatic a or pancreatic ductal epithelial cells.

15. A non-human transgenic mammal comprising at least one inactivated Pax4 allele.

16. The non-human transgenic mammal according to claim 15 further comprising at least one inactivated Pax6 allele.

17. The non-human transgenic animal of claim 15, wherein the at least one inactivated Pax4 allele has been generated by homologous recombination with a construct comprising the β-galactosidase-gene in frame with the amino-terminus of Pax4.

18. The non-human transgenic mammal according to claim 17, which is a mouse or a rat.

19. Use of an effective dose of a nucleic acid sequence encoding a functional and expressible Pax4 protein and optionally a second nucleic acid sequence encoding a functional and expressible Pax6 protein, for the preparation of a pharmaceutical composition for treating, preventing and/or delaying diabetes in a mammal.

20. The use of claim 19, wherein the nucleic acid sequence is operatively linked to regulatory elements allowing for the expression and/or targeting of the Pax4 protein and optionally the Pax6 protein to specific cells.

21. Use of an effective dose of a functional Pax4 protein and optionally a functional Pax6 protein, for the preparation of a pharmaceutical composition for treating, preventing and/or delaying of diabetes in a mammal.

22. The use of any one of claim 19 to 21, wherein said pharmaceutical composition is administered to stimulate the expression of the Pax4 gene and optionally of the Pax6 gene in pancreatic cells and/or to introduce a functional and expressible Pax4 gene and optionally a functional and expressible Pax6 gene in pancreatic cells of a mammal.

23. Use of a functional and expressible Pax4 gene and optionally a functional and expressible Pax6 gene for the preparation of a pharmaceutical composition for the treatment of diabetes in a mammal.

24. The use of claim 23, wherein said pharmaceutical composition is in vitro introduced into a cell of a mammal.

25. The use of clam 24, wherein said cell is to be introduced into said mammal or into a mammal of the same species.

26. The use of claims 24 or 25, wherein said cell is a non-human germ cell, a non-human egg cell, a non-human embryonic cell or a non-human cell derived therefrom.

27. The use of any one of claims 19 to 25, wherein said mammal is a human, or the use of claim 26, wherein said mammal is a rat or mouse.

28. Use of the non-human transgenic animal of claim 15, 17 or 18 for the investigation of diseases associated with a developmental disorder of the pancreas.

29. The use of claim 28, wherein said developmental disorder of the pancreas is juvenile diabetes.

## Patentansprüche

1. In vitro Verfahren zum Testen des Differenzierungsstatus in Bauchspeicheldrüsenzellen eines Säugetiers, umfassend
(a) das Feststellen des Anteils oder Status von Pax4 mRNA in Bauchspeicheldrüsenzellen des genannten Säugetiers; und/oder
(b) das Feststellen des Anteils oder Status von Pax4 Protein in Bauchspeicheldrüsenzellen des genannten Säugetieres; und
(c) das Vergleichen des genannten Anteils oder Status von Pax4 mRNA und/oder Pax4 Protein mit dem entsprechenden Anteil in normalen Bauchspeicheldrüsenzellen,
wobei das genannte Feststellen und der genannte Vergleich des Status von Pax4 mRNA und/oder Pax4 Protein die Feststellung und den Vergleich kennzeichnet, ob die/das genannte mRNA oder Protein eine Mutation, Deletion oder eine posttranslationale Modifikation trägt, was die Gesamtaktivität des Gens bei einem Vergleich mit dem normalen Genprodukt des Wildtyps beeinflussen würde.

2. Verfahren nach Anspruch 1, weiterhin umfassend
(d) das Feststellen des Anteils oder Status von Pax6 mRNA in Bauchspeicheldrüsenzellen des genannten Säugetiers; und/oder
(e) das Feststellen des Anteils oder Status von Pax6 Protein in Bauchspeicheldrüsenzellen des genannten Säugetiers; und
(f) den Vergleich des genannten Anteils oder Status von Pax6 mRNA und/oder Pax6 Protein mit dem entsprechenden Anteil in normalen Bauchspeicheldrüsenzellen,
wobei das genannte Feststellen und der Vergleich des Status von Pax6 mRNA und/oder Pax6 Protein die Feststellung und den Vergleich kennzeichnet, ob die/das genannte mRNA oder Protein eine Mutation, Deletion oder eine posttranslationale Modifikation trägt, was die Gesamtaktivität des Gens bei einem Vergleich mit dem normalen Genprodukt des Wildtyps beeinflussen würde.

3. Verfahren nach Anspruch 1 oder 2, wobei sich das Säugetier in der
(i) embryonalen;
(ii) neugeborenen; oder
(iii) adulten Phase befindet.

4. Verfahren nach einem der Ansprüche 1 bis 3, wobei es sich bei dem genannten Säugetier um eine Maus handelt, oder wobei es sich bei dem genannten Säugetier um einen Menschen handelt.

5. Verfahren nach einem der Ansprüche 1 bis 4, wobei das genannte Feststellen in Schritt (a) und wahlweise in Schritt (d) durch den Einsatz
(i) einer Nukleinsäuresequenz, die mindestens einem Teil des Pax4-Gens entspricht und bevorzugt für mindestens einen Teil des Pax4 Proteins kodiert, und wahlweise einer zweiten Nukleinsäuresequenz, die mindestens einem Teil des Pax6-Gens entspricht und bevorzugt für mindestens einen Teil des Pax6 Proteins kodiert;
(ii) einer Nukleinsäuresequenz komplementär zu der (den) Nukleinsäuresequenz(en) von (i); oder
(iii) eines Starters oder eines Starterpaares, das an die Nukleinsäuresequenz(en) von (i) oder (ii) hybridisiert, erfolgt.

6. Verfahren nach einem der Ansprüche 1 bis 4, wobei das genannte Feststellen in Schritt (b), und wahlweise in Schritt (e), durch den Einsatz eines Antikörpers oder eines Fragments desselben erfolgt, der sich speziell an das Pax4 Protein bindet, und wahlweise durch den Einsatz eines zweiten Antikörpers oder eines Fragments desselben, der sich speziell an das Pax6 Protein bindet.

7. Verfahren nach einem der Ansprüche 1 bis 6, wobei es sich bei den genannten Bauchspeicheldrüsenzellen um β-Zellen oder δ-Zellen handelt.

8. Verfahren nach einem der Ansprüche 1 bis 7, wobei der genannte Entwicklungsstatus auf die Malignität oder das maligne Potenzial der genannten Bauchspeicheldrüsenzelle hinweist.

9. Verfahren nach einem der Ansprüche 1 bis 7, wobei der genannte Differenzierungsstatus in den genannten β-Zellen auf juvenile Diabetes hinweist.

10. Verfahren nach Anspruch 9, wobei der Differenzierungsstatus in den genannten β-Zellen im embryonalen Status oder in dem neugeborenen Status ausgeführt wird.

11. Verfahren nach einem der Ansprüche 1 bis 10, wobei der genannte Differenzierungsstatus das Ergebnis der Aktivität eines Medikamentes oder eines Gentherapieansatzes ist.

12. Verfahren nach Anspruch 11, wobei das genannte Medikament den Expressionsanteil des Pax4-Gens, und wahlweise des Pax6-Gens am mRNA- oder Proteinebene beeinflusst.

13. Verfahren nach einem der Ansprüche 1 bis 10, wobei der genannte Differenzierungsstatus in Pax4-Knock-out-Mäusen getestet wird, die wahlweise gleichzeitig auch Pax6-Knock-out-Mäuse sind.

14. Verfahren nach einem der Ansprüche 1 bis 10, weiterhin umfassend das Einführen des funktionellen und exprimierbaren Pax4-Gens, und wahlweise weiterhin umfassend das Einführen eines funktionellen und exprimierbaren Pax6-Gens in die Bauchspeicheldrüsen-alpha-Zellen oder Bauchspeicheldrüsenkanal-Epithelzellen.

15. Nicht zur menschlichen Rasse gehörendes, transgenes Säugetier, umfassend mindestens ein inaktiviertes Pax4-Allel.

16. Nicht zur menschlichen Rasse gehörendes, transgenes Säugetier gemäß Anspruch 15, weiterhin umfassend mindestens ein inaktiviertes Pax6-Allel.

17. Nicht zur menschlichen Rasse gehörendes, transgenes Säugetier gemäß Anspruch 15, wobei mindestens ein inaktiviertes Pax4-Allel durch homologe Rekombination mit einem Konstrukt umfassend das β-Galactosidase-Gen im Leserahmen des Aminoterminus von Pax4 erzeugt worden ist.

18. Nicht zur menschlichen Rasse gehörendes, transgenes Säugetier gemäß Anspruch 17, wobei es sich dabei um eine Maus oder eine Ratte handelt.

19. Verwendung einer effektiven Nukleinsäuresequenzdosis, die für ein funktionelles und exprimierbares Pax4 Protein kodiert, und wahlweise eine zweite Nukleinsäuresequenzdosis, die für ein funktionelles und exprimierbares Pax6 Protein kodiert, zur Herstellung einer pharmazeutischen Zusammensetzung zur Behandlung, Verhütung und/oder Verzögerung von Diabetes in einem Säugetier.

20. Verwendung nach Anspruch 19, wobei die Nukleinsäuresequenz mit den regulatorischen Elementen funktionell verbunden ist, wodurch die Expression und/oder Zielsetzung des Pax4 Proteins, und wahlweise des Pax6 Proteins, für spezifische Zellen ermöglicht wird.

21. Verwendung einer effektiven Dosis eines funktionellen Pax4 Proteins, und wahlweise eines funktionellen Pax6 Proteins, zur Herstellung einer pharmazeutischen Zusammensetzung zur Behandlung, Verhütung und/oder Verzögerung von Diabetes in einem Säugetier.

22. Verwendung nach einem der Ansprüche 19 bis 21, wobei die genannte pharmazeutische Zusammensetzung verabreicht wird, um die Expression des Pax4-Gens, und wahlweise des Pax6-Gens, in Bauchspeicheldrüsenzellen zu stimulieren und/oder ein funktionelles und exprimierbares Pax4-Gen, und wahlweise ein funktionelles und exprimierbares Pax6―Gen, in Bauchspeicheldrüsenzellen eines Säugetiers einzuführen.

23. Verwendung eines funktionellen und exprimierbaren Pax4-Gens, und wahlweise eines funktionellen und exprimierbaren Pax6-Gens, zur Herstellung einer pharmazeutischen Zusammensetzung zur Behandlung von Diabetes in einem Säugetier.

24. Verwendung nach Anspruch 23, wobei die genannte pharmazeutische Zusammensetzung in vitro in eine Zelle eines Säugetiers eingeführt wird.

25. Verwendung nach Anspruch 24, wobei die genannte Zelle in das genannte Säugetier, oder in ein Säugetier der gleichen Art eingeführt werden soll.

26. Verwendung nach Anspruch 24 oder 25, wobei es sich bei der genannten Zelle um eine nicht-menschliche Keimzelle, eine nicht-menschliche Eizelle, eine nicht-menschliche Embryozelle oder eine davon abgeleitete, nicht-menschliche Zelle handelt.

27. Verwendung nach einem der Ansprüche 19 bis 25, wobei es sich bei dem Säugetier um einen Menschen handelt, oder die Verwendung nach Anspruch 26, wobei das Säugetier eine Ratte oder eine Maus ist.

28. Verwendung eines nicht-menschlichen, transgenen Tiers gemäß Anspruch 15, 17 oder 18 zur Untersuchung von Krankheiten, die mit einer Entwicklungsstörung der Bauchspeicheldrüse in Zusammenhang stehen.

29. Verwendung nach Anspruch 28, wobei es sich bei der genannten Entwicklungsstörung der Bauchspeicheldrüse um juvenile Diabetes handelt.

## Revendications

1. Procédé *in vitro* pour tester l'état de différenciation des cellules pancréatiques d'un mammifère comprenant :
(a) la détermination du niveau ou de l'état du Pax4 mARN des cellules pancréatiques dudit mammifères ; et/ou
(b) la détermination du niveau ou de l'état de la protéine Pax4 des cellules pancréatiques dudit mammifère ; et
(c) la comparaison dudit niveau ou état du Pax4 mARN et/ou de la protéine Pax4 avec un niveau correspondant présent dans des cellules pancréatiques normales.
selon lequel ladite détermination et comparaison de l'état du Pax4 mARN et/ou de la protéine Pax4 signifie la détermination et la comparaison si ladite mRNA ou protéine comprend une mutation, une délétion ou une modification post-translationnelle qui aurait des répercussions sur l'ensemble de l'activité du gène lorsque comparé au produit normal du gène de type sauvage.

2. Procédé selon la revendication 1, qui comprend en outre :
(d) la détermination du niveau ou de l'état du Pax6 mARN des cellules pancréatiques dudit mammifère ; et/ou
(e) la détermination du niveau ou de l'état de la protéine Pax6 des cellules pancréatiques dudit mammifère ; et
(f) la comparaison dudit niveau ou état du Pax6 mARN et/ou de la protéine Pax6 avec un niveau correspondant présent dans des cellules pancréatiques normales,
selon lequel ladite détermination et comparaison de l'état du Pax6 mARN et/ou de la protéine Pax6 signifie la détermination et la comparaison de la mesure selon laquelle ladite mRNA ou protéine comprend une mutation, une déletion ou une modification post-translationnelle qui aurait des répercussions sur l'ensemble de l'activité du gène lorsque comparé au produit normal du gène de type sauvage.

3. Procédé selon la revendication 1 ou 2, selon lequel ledit mammifère se trouve à l'étape :
(i) embryonnaire
(ii) nouveau-né ; ou
(iii) adulte.

4. Procédé selon l'une quelconque des revendications 1 à 3 selon lequel ledit mammifère est une souris ou selon lequel ledit mammifère est un humain.

5. Procédé selon l'une quelconque des revendications 1 à 4, selon lequel ladite détermination de l'étape (a) et éventuellement de l'étape (d) se trouve affectée par l'utilisation :
(i) d'une séquence d'acide nucléique correspondant à au moins une partie du gène Pax4 et de préférence codant au moins une partie de la protéine Pax4 et éventuellement une deuxième séquence d'acide nucléique correspondant à au moins une partie du gène Pax6 et de préférence codant au moins une partie de la protéine Pax6 ;
(ii) une séquence d'acide nucléique complémentaire à la ou les séquence(s) selon (i) ; ou
(iii) une amorce ou une paire d'amorces hybridée sur la ou les séquence(s) selon (i) ou (ii).

6. Procédé selon l'une quelconque des revendications 1 à 4, selon lequel la détermination de l'étape (b) et éventuellement de l'étape (e) se trouve affectée par l'utilisation d'un anticorps ou d'un fragment de ceci qui se lie spécifiquement à la protéine Pax4 et éventuellement moyennant utilisation d'un deuxième anticorps ou fragment de ceci qui se lie spécifiquement à la protéine Pax6.

7. Procédé selon l'une quelconque des revendications 1 à 6, selon lequel lesdites cellules pancréatiques sont des cellules-β ou des cellules-δ.

8. Procédé selon l'une quelconque des revendications 1 a 7, selon lequel l'état de développement indique une malignité ou un potentiel de malignité de ladite cellule pancréatique.

9. Procédé selon l'une quelconque des revendications 1 à 7, selon lequel ledit état de différentiation desdites cellules-β indique le diabète juvénile.

10. Procédé selon la revendication 9, selon lequel ledit état de différentiation desdites cellules-β se déroule à l'état embryonnaire ou à l'état nouveau-né.

11. Procédé selon l'une quelconque des revendications 1 à 10 selon lequel ledit état de différentiation est issu de l'activité d'un médicament ou d'une approche de thérapie des gènes.

12. Procédé selon la revendication 11, selon lequel le médicament affecte le niveau d'expression du gène pax4 et éventuellement du gène Pax6 au niveau de la mARN ou de la protéine.

13. Procédé selon l'une quelconque des revendications 1 à 10 selon lequel ledit état de différentiation est testé avec des souris Pax4 «knockout» et qui éventuellement sont aussi des souris Pax6 «knockout».

14. Procédé selon l'une quelconque des revendications 1 à 10, comprenant en outre l'introduction du gène Pax4 fonctionnel et exprimable et éventuellement en outre comprenant l'introduction d'un gène Pax6 fonctionnel et exprimable dans des cellules pancréatiques α ou épithéliales canalaires pancréatiques.

15. Mammifère transgénique non-humain comprenant au moins un allèle Pax4 inactivé.

16. Mammifère transgénique non-humain selon la revendication 15 comprenant en outre au moins un allèle Pax6 inactivé.

17. Animal transgénique non-humain de la revendication 15, selon lequel au moins un allèle Pax4 inactivé a été généré moyennant recombinaison homologue avec un gène hybride comprenant le gène galactosidase-β en plan avec la terminaison amino du Pax4.

18. Mammifère transgénique non-humain selon la revendication 17, qui est une souris ou un rat.

19. Utilisation d'une dose efficace de séquence d'acide nucléique qui code une protéine Pax4 fonctionnelle et exprimable et éventuellement une deuxième séquence d'acide nucléique qui code une protéine Pax6 fonctionnelle et exprimable, pour la préparation d'une composition pharmaceutique destinée au traitement, à la prévention et/ou à retarder le diabète chez un mammifère.

20. Utilisation de la revendication 19, selon laquelle la séquence d'acide nucléique est liée opérativement à des éléments régulateurs qui permettent l'expression et/ou le ciblage de la protéine Pax4 et éventuellement de la protéine Pax6 dans des cellules spécifiques.

21. Utilisation d'une dose efficace d'une protéine fonctionnelle Pax4 et éventuellement d'une protéine Pax6 fonctionnelle, pour la préparation d'une composition pharmaceutique destinée au traitement, à la prévention et/ou à retarder le diabète chez un mammifère.

22. Utilisation de l'une quelconque des revendications 19 à 21, selon laquelle la dite composition pharmaceutique est administrée pour stimuler l'expression du gène Pax4 et éventuellement du gène Pax6 dans les cellules pancréatiques et/ou pour introduire un gène Pax4 fonctionnel et exprimable et éventuellement un gène Pax6 fonctionnel et exprimable dans les cellules pancréatiques d'un mammifère.

23. Utilisation d'un gène Pax4 fonctionnel et exprimable et éventuellement d'un gène Pax6 fonctionnel et exprimable pour la préparation d'une composition pharmaceutique destinée au traitement du diabète chez un mammifère.

24. Utilisation de la revendication 23, selon laquelle ladite composition pharmaceutique est introduite *in vitro* dans la cellule d'un mammifère.

25. Utilisation de la revendication 24, selon laquelle ladite cellule sera introduite dans ledit mammifère ou dans un mammifère de la même espèce.

26. Utilisation de la revendication 24 ou 25, selon laquelle ladite cellule est une cellule germinale non-humaine, une cellule ovocyte non-humaine, une cellule embryonnaire non-humaine, ou une cellule dérivée non-humaine dérivé de ceci.

27. Utilisation de l'une quelconque des revendications 19 à 25, selon laquelle ledit mammifère est un humain, ou utilisation da la revendication 26, selon laquelle ledit mammifère est un rat ou une souris.

28. Utilisation de l'animal transgénique non-humain de la la revendication 15, 17, ou 18 pour la recherche des maladies associées aux troubles du développement du pancréas.

29. Utilisation de la revendication 28, selon laquelle le trouble du développement du pancréas est le diabète juvénile.


### SEQUENCE LISTING

(1) GENERAL INFORMATION:
   (i) APPLICANT:
      (A) NAME: Max-Planck-Gesellschaft zur Förderung der Wissenschaften e.V.
      (B) STREET: none
      (C) CITY: Berlin
      (E) COUNTRY: Germany
      (F) POSTAL CODE (ZIP): none
   (ii) TITLE OF INVENTION: NOVEL METHOD FOR TESTING THE DIFFERENTIATION STATUS IN PANCREATIC CELLS OF A MAMMAL
   (iii) NUMBER OF SEQUENCES: 18
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30 (EPO)
(2) INFORMATION FOR SEQ ID NO: 1:
   (i) SEQUENCE CHARACTERISTICS:
      (A) LENGTH: 1275 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (ix) FEATURE:
      (A) NAME/KEY: CDS
      (B) LOCATION:156..1161
   (xi) SEQUENCE DESCRIPTION: SEQ ID NO: 1:
   ACCAGCAACC CTGGAGCCTG CACAGACCCT GAGACCTCTT CCTGAATTCC CACCTTTTTT  60
   CCTCCATCCA GTACCAGTCC CAAAGAGAAA CTTCCAGAAG GAGCTCTCCG TTTTCAGTTT 120
   GCCAGTTGGC TTCCTGTCCT TCTGCGAGGA GTACCAGTGT GAAGC ATG CAG CAG     174
                                                     Met Gln Gln
                                                      1
   GAC GGA CTC AGC AGT GTG AAT CAG CTA GGG GGA CTC TTT GTG AAT GGC
   222
   Asp Gly Leu Ser Ser Val Asn Gln Leu Gly Gly Leu Phe Val Asn Gly
        5                  10                  15
   CGG CCC CTT CCT CTG GAC ACC AGG CAG CAG ATT GTG CAG CTA GCA ATA 270
   Arg Pro Leu Pro Leu Asp Thr Arg Gln Gln Ile Val Gln Leu Ala Ile
   20 25 30 35
   AGA GGG ATG CGA CCC TGT GAC ATT TCA CGG AGC CTT AAG GTA TCT AAT 318
   Arg Gly Met Arg Pro Cys Asp Ile Ser Arg Ser Leu Lys Val Ser Asn
   40 45 50
   GGC TGT GTG AGC AAG ATC CTA GGA CGC TAC TAC CGC ACA GGT GTC TTG 366
   Gly Cys Val Ser Lys Ile Leu Gly Arg Tyr Tyr Arg Thr Gly Val Leu
   55 60 65
   GAA CCC AAG TGT ATT GGG GGA AGC AAA CCA CGT CTG GCC ACA CCT GCT 414
   Glu Pro Lys Cys Ile Gly Gly Ser Lys Pro Arg Leu Ala Thr Pro Ala
   70 75 80
   GTG GTG GCT CGA ATT GCC CAG CTA AAG GAT GAG TAC CCT GCT CTT TTT 462
   Val Val Ala Arg Ile Ala Gln Leu Lys Asp Glu Tyr Pro Ala Leu Phe
   85 90 95
   GCC TGG GAG ATC CAA CAC CAG CTT TGC ACT GAA GGG CTT TGT ACC CAG 510
   Ala Trp Glu Ile Gln His Gln Leu Cys Thr Glu Gly Leu Cys Thr Gln
   100 105 110 115
   GAC AAG GCT CCC AGT GTG TCC TCT ATC AAT CGA GTA CTT CGG GCA CTT 558
   Asp Lys Ala Pro Ser Val Ser Ser Ile Asn Arg Val Leu Arg Ala Leu
   120 125 130
   CAG GAA GAC CAG AGC TTG CAC TGG ACT CAA CTC AGA TCA CCA GCT GTG 606
   Gln Glu Asp Gln Ser Leu His Trp Thr Gln Leu Arg Ser Pro Ala Val
   135 140 145
   TTG GCT CCA GTT CTT CCC AGT CCC CAC AGT AAC TGT GGG GCT CCC CGA 654
   Leu Ala Pro Val Leu Pro Ser Pro His Ser Asn Cys Gly Ala Pro Arg
   150 155 160
   GGC CCC CAC CCA GGA ACC AGC CAC AGG AAT CGG GCT ATC TTC TCC CCG 702
   Gly Pro His Pro Gly Thr Ser His Arg Asn Arg Ala Ile Phe Ser Pro
   165 170 175
   GGA CAA GCC GAG GCA CTG GAG AAA GAG TTT CAG CGT GGG CAG TAT CCA    750
   Gly Gln Ala Glu Ala Leu Glu Lys Glu Phe Gln Arg Gly Gln Tyr Pro
   180                 185                 190                 195
   GAT TCA GTG GCC CGT GGG AAG CTG GCT GCT GCC ACC TCT CTG CCT GAA    798
   Asp Ser Val Ala Arg Gly Lys Leu Ala Ala Ala Thr Ser Leu Pro Glu
               200                 205                 210
   GAC ACG GTG AGG GTT TGG TTT TCT AAC AGA AGA GCC AAA TGG CGC AGG    846
   Asp Thr Val Arg Val Trp Phe Ser Asn Arg Arg Ala Lys Trp Arg Arg
           215                 220                 225
   CAA GAG AAG CTG AAA TGG GAA GCA CAG CTG CCA GGT GCT TCC CAG GAC    894
   Gln Glu Lys Leu Lys Trp Glu Ala Gln Leu Pro Gly Ala Ser Gln Asp
       230                 235                 240
   CTG ACG ATA CCA AAA AAT TCT CCA GGG ATC ATC TCT GCA CAG CAG TCC    942
   Leu Thr Ile Pro Lys Asn Ser Pro Gly Ile Ile Ser Ala Gln Gln Ser
       245                 250                 255
   CCC GGC AGT GTA CCC TCA GCT GCC TTG CCT GTG CTG GAA CCA TTG AGT    990
   Pro Gly Ser Val Pro Ser Ala Ala Leu Pro Val Leu Glu Pro Leu Ser
   260                 265                 270                 275
   CCT TCC TTC TGT CAG CTA TGC TGT GGG ACA GCA CCA GGC AGA TGT TCC   1038
   Pro Ser Phe Cys Gln Leu Cys Cys Gly Thr Ala Pro Gly Arg Cys Ser
               280                 285                 290
   AGT GAC ACC TCA TCC CAG GCC TAT CTC CAA CCC TAC TGG GAC TGC CAA   1086
   Ser Asp Thr Ser Ser Gln Ala Tyr Leu Gln Pro Tyr Trp Asp Cys Gln
           295                 300                 305
   TCC CTC CTT CCT GTG GCT TCC TCC TCA TAT GTG GAA TTT GCC TGC CCT   1134
   Ser Leu Leu Pro Val Ala Ser Ser Ser Tyr Val Glu Phe Ala Cys Pro
       310                 315                 320
   GCC TCA CCA CCC ATC CTG TGC ATC ATC TGATTGGAGG CCCAGGACAA         1181
   Ala Ser Pro Pro Ile Leu Cys Ile Ile
       325                 330
   GTGCCATCAT CCCATTGCTC AAACTGGCCA TAAGACACCT CTATTTGACA GTAATAAAAA
   1241
   CCTTTTCTTA GATGTTAAAA AAAAAAAAGG GGGG
   1275
(2) INFORMATION FOR SEQ ID NO: 2:
   (i) SEQUENCE CHARACTERISTICS:
      (A) LENGTH: 332 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Gln Gln Asp Gly Leu Ser Ser Val Asn Gln Leu Gly Gly Leu Phe
   1 5 10 15
   Val Asn Gly Arg Pro Leu Pro Leu Asp Thr Arg Gln Gln Ile Val Gln
   20 25 30
   Leu Ala Ile Arg Gly Met Arg Pro Cys Asp Ile Ser Arg Ser Leu Lys
   35 40 45
   Val Ser Asn Gly Cys Val Ser Lys Ile Leu Gly Arg Tyr Tyr Arg Thr
   50 55 60
   Gly Val Leu Glu Pro Lys Cys Ile Gly Gly Ser Lys Pro Arg Leu Ala
   65 70 75 80
   Thr Pro Ala Val Val Ala Arg Ile Ala Gln Leu Lys Asp Glu Tyr Pro
   85 90 95
   Ala Leu Phe Ala Trp Glu Ile Gln His Gln Leu Cys Thr Glu Gly Leu
   100 105 110
   Cys Thr Gln Asp Lys Ala Pro Ser Val Ser Ser Ile Asn Arg Val Leu
   115 120 125
   Arg Ala Leu Gln Glu Asp Gln Ser Leu His Trp Thr Gln Leu Arg Ser
   130 135 140
   Pro Ala Val Leu Ala Pro Val Leu Pro Ser Pro His Ser Asn Cys Gly
   145 150 155 160
   Ala Pro Arg Gly Pro His Pro Gly Thr Ser His Arg Asn Arg Ala Ile
   165 170 175
   Phe Ser Pro Gly Gln Ala Glu Ala Leu Glu Lys Glu Phe Gln Arg Gly
   180 185 190
   Gln Tyr Pro Asp Ser Val Ala Arg Gly Lys Leu Ala Ala Ala Thr Ser
   195 200 205
   Leu Pro Glu Asp Thr Val Arg Val Trp Phe Ser Asn Arg Arg Ala Lys
   210 215 220
   Trp Arg Arg Gln Glu Lys Leu Lys Trp Glu Ala Gln Leu Pro Gly Ala
   225 230 235 240
   Ser Gln Asp Leu Thr Ile Pro Lys Asn Ser Pro Gly Ile Ile Ser Ala
   245 250 255
   Gln Gln Ser Pro Gly Ser Val Pro Ser Ala Ala Leu Pro Val Leu Glu
   260 265 270
   Pro Leu Ser Pro Ser Phe Cys Gln Leu Cys Cys Gly Thr Ala Pro Gly
   275 280 285
   Arg Cys Ser Ser Asp Thr Ser Ser Gln Ala Tyr Leu Gln Pro Tyr Trp
   290 295 300
   Asp Cys Gln Ser Leu Leu Pro Val Ala Ser Ser Ser Tyr Val Glu Phe
   305 310 315 320
   Ala Cys Pro Ala Ser Pro Pro Ile Leu Cys Ile Ile
   325 330
(2) INFORMATION FOR SEQ ID NO: 3:
   (i) SEQUENCE CHARACTERISTICS:
      (A) LENGTH: 2481 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (ix) FEATURE:
      (A) NAME/KEY: CDS
      (B) LOCATION:163..1470
   (xi) SEQUENCE DESCRIPTION: SEQ ID NO: 3:
   ACAACGACGA AAGAGAGGAT GCCTCTTAAA GGCAGAAGAC TTTAACCAAG GGCGGTGAGC  60
   AGATGTGTGA GATCTTCTAT TCTAGAAGTG GACGTATATC CCAGTTCTCA GAGCCCCGTA 120
   TTCGAGCCCC GTGGGATCCG GAGGCTGCCA ACCAGCTCCA GC ATG CAG AAC AGT   174
                                                  Met Gln Asn Ser
                                                   1
   CAC AGC GGA GTG AAT CAG CTT GGT GGT GTC TTT GTC AAC GGG CGG CCA   222
   His Ser Gly Val Asn Gln Leu Gly Gly Val Phe Val Asn Gly Arg Pro
     5                  10                  15                  20
   CTG CCG GAC TCC ACC CGG CAG AAG ATC GTA GAG CTA GCT CAC AGC GGG   270
   Leu Pro Asp Ser Thr Arg Gln Lys Ile Val Glu Leu Ala His Ser Gly
                    25                  30                  35
   GCC CGG CCG TGC GAC ATT TCC CGA ATT CTG CAG ACC CAT GCA GAT GCA   318
   Ala Arg Pro Cys Asp Ile Ser Arg Ile Leu Gln Thr His Ala Asp Ala
                40                  45                  50
   AAA GTC CAG GTG CTG GAC AAT GAA AAC GTA TCC AAC GGT TGT GTG AGT   366
   Lys Val Gln Val Leu Asp Asn Glu Asn Val Ser Asn Gly Cys Val Ser
            55                  60                  65
   AAA ATT CTG GGC AGG TAT TAC GAG ACT GGC TCC ATC AGA CCC AGG GCA   414
   Lys Ile Leu Gly Arg Tyr Tyr Glu Thr Gly Ser Ile Arg Pro Arg Ala
        70                  75                  80
   ATC GGA GGG AGT AAG CCA AGA GTG GCG ACT CCA GAA GTT GTA AGC AAA   462
   Ile Gly Gly Ser Lys Pro Arg Val Ala Thr Pro Glu Val Val Ser Lys
    85                  90                  95                 100
   ATA GCC CAG TAT AAA CGG GAG TGC CCT TCC ATC TTT GCT TGG GAA ATC   510
   Ile Ala Gln Tyr Lys Arg Glu Cys Pro Ser Ile Phe Ala Trp Glu Ile
                   105                 110                 115
   CGA GAC AGA TTA TTA TCC GAG GGG GTC TGT ACC AAC GAT AAC ATA CCC   558
   Arg Asp Arg Leu Leu Ser Glu Gly Val Cys Thr Asn Asp Asn Ile Pro
               120                 125                 130
   AGT GTG TCA TCA ATA AAC AGA GTT CTT CGC AAC CTG GCT AGC GAA AAG   606
   Ser Val Ser Ser Ile Asn Arg Val Leu Arg Asn Leu Ala Ser Glu Lys
           135                 140                 145
   CAA CAG ATG GGC GCA GAC GGC ATG TAT GAT AAA CTA AGG ATG TTG AAC    654
   Gln Gln Met Gly Ala Asp Gly Met Tyr Asp Lys Leu Arg Met Leu Asn
       150                 155                 160
   GGG CAG ACC GGA AGC TGG GGC ACA CGC CCT GGT TGG TAT CCC GGG ACT    702
   Gly Gln Thr Gly Ser Trp Gly Thr Arg Pro Gly Trp Tyr Pro Gly Thr
   165                 170                 175                 180
   TCA GTA CCA GGG CAA CCC ACG CAA GAT GGC TGC CAG CAA CAG GAA GGA    750
   Ser Val Pro Gly Gln Pro Thr Gln Asp Gly Cys Gln Gln Gln Glu Gly
                   185                 190                 195
   GGG GGA GAG AAC ACC AAC TCC ATC AGT TCT AAC GGA GAA GAC TCG GAT    798
   Gly Gly Glu Asn Thr Asn Ser Ile Ser Ser Asn Gly Glu Asp Ser Asp
               200                 205                 210
   GAA GCT CAG ATG CGA CTT CAG CTG AAG CGG AAG CTG CAA AGA AAT AGA    846
   Glu Ala Gln Met Arg Leu Gln Leu Lys Arg Lys Leu Gln Arg Asn Arg
           215                 220                 225
   ACA TCT TTT ACC CAA GAG CAG ATT GAG GCT CTG GAG AAA GAG TTT GAG    894
   Thr Ser Phe Thr Gln Glu Gln Ile Glu Ala Leu Glu Lys Glu Phe Glu
       230                 235                 240
   AGG ACC CAT TAT CCA GAT GTG TTT GCC CGG GAA AGA CTA GCA GCC AAA    942
   Arg Thr His Tyr Pro Asp Val Phe Ala Arg Glu Arg Leu Ala Ala Lys
   245                 250                 255                 260
   ATA GAT CTA CCT GAA GCA AGA ATA CAG GTA TGG TTT TCT AAT CGA AGG    990
   Ile Asp Leu Pro Glu Ala Arg Ile Gln Val Trp Phe Ser Asn Arg Arg
                   265                 270                 275
   GCC AAA TGG AGA AGA GAA GAG AAA CTG AGG AAC CAG AGA AGA CAG GCC   1038
   Ala Lys Trp Arg Arg Glu Glu Lys Leu Arg Asn Gln Arg Arg Gln Ala
               280                 285                 290
   AGC AAC ACT CCT AGT CAC ATT CCT ATC AGC AGC AGC TTC AGT ACC AGT   1086
   Ser Asn Thr Pro Ser His Ile Pro Ile Ser Ser Ser Phe Ser Thr Ser
           295                 300                 305
   GTC TAC CAG CCA ATC CCA CAG CCC ACC ACA CCT GTC TCC TCC TTC ACA   1134
   Val Tyr Gln Pro Ile Pro Gln Pro Thr Thr Pro Val Ser Ser Phe Thr
       310                 315                 320
   TCA GGT TCC ATG TTG GGC CGA ACA GAC ACC GCC CTC ACC AAC ACG TAC 1182
   Ser Gly Ser Met Leu Gly Arg Thr Asp Thr Ala Leu Thr Asn Thr Tyr
   325 330 335 340
   AGT GCT TTG CCA CCC ATG CCC AGC TTC ACC ATG GCA AAC AAC CTG CCT 1230
   Ser Ala Leu Pro Pro Met Pro Ser Phe Thr Met Ala Asn Asn Leu Pro
   345 350 355
   ATG CAA CCC CCA GTC CCC AGT CAG ACC TCC TCA TAC TCG TGC ATG CTG 1278
   Met Gln Pro Pro Val Pro Ser Gln Thr Ser Ser Tyr Ser Cys Met Leu
   360 365 370
   CCC ACC AGC CCG TCA GTG AAT GGG CGG AGT TAT GAT ACC TAC ACC CCT 1326
   Pro Thr Ser Pro Ser Val Asn Gly Arg Ser Tyr Asp Thr Tyr Thr Pro
   375 380 385
   CCG CAC ATG CAA ACA CAC ATG AAC AGT CAG CCC ATG GGC ACC TCG GGG 1374
   Pro His Met Gln Thr His Met Asn Ser Gln Pro Met Gly Thr Ser Gly
   390 395 400
   ACC ACT TCA ACA GGA CTC ATT TCA CCT GGA GTG TCA GTT CCC GTC CAA 1422
   Thr Thr Ser Thr Gly Leu Ile Ser Pro Gly Val Ser Val Pro Val Gln
   405 410 415 420
   GTT CCC GGG AGT GAA CCT GAC ATG TCT CAG TAC TGG CCT CGA TTA CAG 1470
   Val Pro Gly Ser Glu Pro Asp Met Ser Gln Tyr Trp Pro Arg Leu Gln
   425 430 435
   TAAAGAGAGA AGGAGAGAGC ATGTGATCGA GAGAGGAAAT TGTGTTCACT CTGCCAATGA
   1530
   CTATGTGGAC ACAGCAGTTG GGTATTCAGG AAAGAAAGAG AAATGGCGGT TAGAAGCACT
   1590
   TCACTTTGTA ACTGTCCTGA ACTGGAGCCC GGGAATGGAC TAGAACCAAG GACCTTGCGT
   1650
   ACAGAAGGCA CGGTATCAGT TGGAACAAAT CTTCATTTTG GTATCCAAAC TTTTATTCAT
   1710
   TTTGGTGTAT TATTTGTAAA TGGGCATTGG TATGTTATAA TGAAGAAAAG AACAACACAG
   1770
   GCTGTTGGAT CGCGGATCTG TGTTGCTCAT GTGGTTGTTT AAAGGAAACC ATGATCGACA 1830
   AGATTTGCCA TGGATTTAAG AGTTTTATCA AGATATATCA AATACTTCTC CCCATCTGTT 1890
   CATAGTTTAT GGACTGATGT TCCAAGTTTG TATCATTCCT TTGCATATAA TTGAACCTGG 1950
   GACAACACAC ACTAGATATA TGTAAAAACT ATCTGTTGGT TTTCCAAAGG TTGTTAACAG 2010
   ATGAAGTTTA TGTGCAAAAA AGGGTAAGAT ATGAATTCAA GGAGAAGTTG ATAGCTAAAA 2070
   GGTAGAGTGT GTCTTCGATA TAATACAATT TGTTTTATGT CAAAATGTAA GTATTTGTCT 2130
   TCCCTAGAAA TCCTCAGAAT GATTTCTATA ATAAAGTTAA TTTCATTTAT ATTTGACAAG 2190
   AATACTCTAT AGATGTTTTA TACACATTTT CATGCAATCA TTTGTTTCTT TCTTGGCCAG 2250
   CAAAAGTTAA TTGTTCTTAG ATATAGCTGT ATTACTGTTC ACAGTCCAAT CATTTTGTGC 2310
   ATCTAGAATT CATTCCTAAT CAATTAAAAG TGCTTGCAAG AGTTTTAAAC CTAAGTGTTT 2370
   TGCAGTTGTT CACAAATACA TATCAAAATT AACCATTGTT GATTGTAAAA AAAAAACCAT 2430
   GCCAAAGCCT TTGTATTTTC TTTATTACCC TTGACCGTAA GACATGAATT C 2481
(2) INFORMATION FOR SEQ ID NO: 4:
   (i) SEQUENCE CHARACTERISTICS:
      (A) LENGTH: 436 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Met Gln Asn Ser His Ser Gly Val Asn Gln Leu Gly Gly Val Phe Val
   1 5 10 15
   Asn Gly Arg Pro Leu Pro Asp Ser Thr Arg Gln Lys Ile Val Glu Leu
   20 25 30
   Ala His Ser Gly Ala Arg Pro Cys Asp Ile Ser Arg Ile Leu Gln Thr
   35 40 45
   His Ala Asp Ala Lys Val Gln Val Leu Asp Asn Glu Asn Val Ser Asn
   50 55 60
   Gly Cys Val Ser Lys Ile Leu Gly Arg Tyr Tyr Glu Thr Gly Ser Ile
   65 70 75 80
   Arg Pro Arg Ala Ile Gly Gly Ser Lys Pro Arg Val Ala Thr Pro Glu
   85 90 95
   Val Val Ser Lys Ile Ala Gln Tyr Lys Arg Glu Cys Pro Ser Ile Phe
   100 105 110
   Ala Trp Glu Ile Arg Asp Arg Leu Leu Ser Glu Gly Val Cys Thr Asn
   115 120 125
   Asp Asn Ile Pro Ser Val Ser Ser Ile Asn Arg Val Leu Arg Asn Leu
   130 135 140
   Ala Ser Glu Lys Gln Gln Met Gly Ala Asp Gly Met Tyr Asp Lys Leu
   145 150 155 160
   Arg Met Leu Asn Gly Gln Thr Gly Ser Trp Gly Thr Arg Pro Gly Trp
   165 170 175
   Tyr Pro Gly Thr Ser Val Pro Gly Gln Pro Thr Gln Asp Gly Cys Gln
   180 185 190
   Gln Gln Glu Gly Gly Gly Glu Asn Thr Asn Ser Ile Ser Ser Asn Gly
   195 200 205
   Glu Asp Ser Asp Glu Ala Gln Met Arg Leu Gln Leu Lys Arg Lys Leu
   210 215 220
   Gln Arg Asn Arg Thr Ser Phe Thr Gln Glu Gln Ile Glu Ala Leu Glu
   225 230 235 240
   Lys Glu Phe Glu Arg Thr His Tyr Pro Asp Val Phe Ala Arg Glu Arg
   245 250 255
   Leu Ala Ala Lys Ile Asp Leu Pro Glu Ala Arg Ile Gln Val Trp Phe
   260 265 270
   Ser Asn Arg Arg Ala Lys Trp Arg Arg Glu Glu Lys Leu Arg Asn Gln
   275 280 285
   Arg Arg Gln Ala Ser Asn Thr Pro Ser His Ile Pro Ile Ser Ser Ser
   290 295 300
   Phe Ser Thr Ser Val Tyr Gln Pro Ile Pro Gln Pro Thr Thr Pro Val
   305 310 315 320
   Ser Ser Phe Thr Ser Gly Ser Met Leu Gly Arg Thr Asp Thr Ala Leu
   325 330 335
   Thr Asn Thr Tyr Ser Ala Leu Pro Pro Met Pro Ser Phe Thr Met Ala
   340 345 350
   Asn Asn Leu Pro Met Gln Pro Pro Val Pro Ser Gln Thr Ser Ser Tyr
   355 360 365
   Ser Cys Met Leu Pro Thr Ser Pro Ser Val Asn Gly Arg Ser Tyr Asp
   370 375 380
   Thr Tyr Thr Pro Pro His Met Gln Thr His Met Asn Ser Gln Pro Met
   385 390 395 400
   Gly Thr Ser Gly Thr Thr Ser Thr Gly Leu Ile Ser Pro Gly Val Ser
   405 410 415
   Val Pro Val Gln Val Pro Gly Ser Glu Pro Asp Met Ser Gln Tyr Trp
   420 425 430
   Pro Arg Leu Gln
   435
(2) INFORMATION FOR SEQ ID NO: 5:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 5:
   AGCAATAAGA GGGATGCGAC C 21
(2) INFORMATION FOR SEQ ID NO: 6:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 6:
   AGCTGTGCTT CCCATTTCAG C 21
(2) INFORMATION FOR SEQ ID NO: 7:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 7:
   GCGAATTCCC TGAAGTGCCC GAAGTACTCG ATT 33
(2) INFORMATION FOR SEQ ID NO: 8:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 8:
   GGCTCCGTGA AATGTCACAG 20
(2) INFORMATION FOR SEQ ID NO: 9:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 9:
(2) INFORMATION FOR SEQ ID NO: 10:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 10:
   CGTAGTACTG TCGACTAGCA 20
(2) INFORMATION FOR SEQ ID NO: 11:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 11:
   AACTGGAAGA ATTCGCGGCC GCAGGAA 27
(2) INFORMATION FOR SEQ ID NO: 12:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 12:
   CAGGAAGACC AGAGCTTGCA CTGG 24
(2) INFORMATION FOR SEQ ID NO: 13:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 13:
   GCGGATCCCA CAGGAATCGG GCTATCTTC 29
(2) INFORMATION FOR SEQ ID NO: 14:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 14:
   GCGCAGGCAA GAGAAGCTGA 20
(2) INFORMATION FOR SEQ ID NO: 15:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 15:
   CTTCCAGAAG GAGCTCTC 18
(2) INFORMATION FOR SEQ ID NO: 16:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 16:
   TGGGATGATG GCACTTGTC 19
(2) INFORMATION FOR SEQ ID NO: 17:
   (i) SEQUENCE CHARACTERISTICS:
      (A) LENGTH: 1280 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (ix) FEATURE:
      (A) NAME/KEY: CDS
      (B) LOCATION:168..1214
   (xi) SEQUENCE DESCRIPTION: SEQ ID NO: 17:
   CCACCAGCAA CCCTGGAGCC TGCACAGACC CTGAGACCTC TTCCTGAATT CCCACCTTTT  60
   TTCCTCCATC CAGTACCAGT CCCAAAGAGA AACTTCCAGA AGGAGCTCTC CGTTTTCAGT 120
   TTGCCAGTTG GCTTCCTGTC CTTCTGCGAG GAGTACCAGT GTGAAGC ATG CAG CAG   176
                                                       Met Gln Gln
                                                        1
   GAC GGA CTC AGC AGT GTG AAT CAG CTA GGG GGA CTC TTT GTG AAT GGC   224
   Asp Gly Leu Ser Ser Val Asn Gln Leu Gly Gly Leu Phe Val Asn Gly
            5                  10                  15
   CGG CCC CTT CCT CTG GAC ACC AGG CAG CAG ATT GTG CAG CTA GCA ATA   272
   Arg Pro Leu Pro Leu Asp Thr Arg Gln Gln Ile Val Gln Leu Ala Ile
    20                  25                  30                  35
   AGA GGG ATG CGA CCC TGT GAC ATT TCA CGG AGC CTT AAG GTA TCT AAT 320
   Arg Gly Met Arg Pro Cys Asp Ile Ser Arg Ser Leu Lys Val Ser Asn
   40 45 50
   GGC TGT GTG AGC AAG ATC CTA GGA CGC TAC TAC CGC ACA GGT GTC TTG 368
   Gly Cys Val Ser Lys Ile Leu Gly Arg Tyr Tyr Arg Thr Gly Val Leu
   55 60 65
   GAA CCC AAG TGT ATT GGG GGA AGC AAA CCA CGT CTG GCC ACA CCT GCT 416
   Glu Pro Lys Cys Ile Gly Gly Ser Lys Pro Arg Leu Ala Thr Pro Ala
   70 75 80
   GTG GTG GCT CGA ATT GCC CAG CTA AAG GAT GAG TAC CCT GCT CTT TTT 464
   Val Val Ala Arg Ile Ala Gln Leu Lys Asp Glu Tyr Pro Ala Leu Phe
   85 90 95
   GCC TGG GAG ATC CAA CAC CAG CTT TGC ACT GAA GGG CTT TGT ACC CAG 512
   Ala Trp Glu Ile Gln His Gln Leu Cys Thr Glu Gly Leu Cys Thr Gln
   100 105 110 115
   GAC AAG GCT CCC AGT GTG TCC TCT ATC AAT CGA GTA CTT CGG GCA CTT 560
   Asp Lys Ala Pro Ser Val Ser Ser Ile Asn Arg Val Leu Arg Ala Leu
   120 125 130
   CAG GAA GAC CAG AGC TTG CAC TGG ACT CAA CTC AGA TCA CCA GCT GTG 608
   Gln Glu Asp Gln Ser Leu His Trp Thr Gln Leu Arg Ser Pro Ala Val
   135 140 145
   TTG GCT CCA GTT CTT CCC AGT CCC CAC AGT AAC TGT GGG GCT CCC CGA 656
   Leu Ala Pro Val Leu Pro Ser Pro His Ser Asn Cys Gly Ala Pro Arg
   150 155 160
   GGC CCC CAC CCA GGA ACC AGC CAC AGG AAT CGG GCT ATC TTC TCC CCG 704
   Gly Pro His Pro Gly Thr Ser His Arg Asn Arg Ala Ile Phe Ser Pro
   165 170 175
   GGA CAA GCC GAG GCA CTG GAG AAA GAG TTT CAG CGT GGG CAG TAT CCA 752
   Gly Gln Ala Glu Ala Leu Glu Lys Glu Phe Gln Arg Gly Gln Tyr Pro
   180 185 190 195
   GAT TCA GTG GCC CGT GGG AAG CTG GCT GCT GCC ACC TCT CTG CCT GAA 800
   Asp Ser Val Ala Arg Gly Lys Leu Ala Ala Ala Thr Ser Leu Pro Glu
   200 205 210
   GAC ACG GTG AGG GTT TGG TTT TCT AAC AGA AGA GCC AAA TGG CGC AGG 848
   Asp Thr Val Arg Val Trp Phe Ser Asn Arg Arg Ala Lys Trp Arg Arg
   215 220 225
   CAA GAG AAG CTG AAA TGG GAA GCA CAG CTG CCA GGT GCT TCC CAG GAC 896
   Gln Glu Lys Leu Lys Trp Glu Ala Gln Leu Pro Gly Ala Ser Gln Asp
   230 235 240
   CTG ACA GTA CCA AAA AAT TCT CCA GGG ATC ATC TCT GCA CAG CAG TCC 944
   Leu Thr Val Pro Lys Asn Ser Pro Gly Ile Ile Ser Ala Gln Gln Ser
   245 250 255
   CCC GGC AGT GTA CCC TCA GCT GCC TTG CCT GTG CTG GAA CCA TTG AGT 992
   Pro Gly Ser Val Pro Ser Ala Ala Leu Pro Val Leu Glu Pro Leu Ser
   260 265 270 275
   CCT TCC TTC TGT CAG CTA TGC TGT GGG ACA GCA CCA GGC AGA TGT TCC 1040
   Pro Ser Phe Cys Gln Leu Cys Cys Gly Thr Ala Pro Gly Arg Cys Ser
   280 285 290
   AGT GAC ACC TCA TCC CAG GCC TAT CTC CAA CCC TAC TGG GAC TGC CAA 1088
   Ser Asp Thr Ser Ser Gln Ala Tyr Leu Gln Pro Tyr Trp Asp Cys Gln
   295 300 305
   TCC CTC CTT CCT GTG GCT TCC TCC TCA TAT GTG GAA TTT GCC TGG CCC 1136
   Ser Leu Leu Pro Val Ala Ser Ser Ser Tyr Val Glu Phe Ala Trp Pro
   310 315 320
   TGC CTC ACC ACC CAT CCT GTG CAT CAT CTG ATT GGA GGC CCA GGA CAA 1184
   Cys Leu Thr Thr His Pro Val His His Leu Ile Gly Gly Pro Gly Gln
   325 330 335
   GTG CCA TCA ACC CAT TGC TCA AAC TGG CCA TAAGAGGCCT NTATTTGACA 1234
   Val Pro Ser Thr His Cys Ser Asn Trp Pro
   340 345
   GTAATAAAAA CCTTTTTTTA GATGTTAAAA AAAAAAAAAA AAAAAA 1280
(2) INFORMATION FOR SEQ ID NO: 18:
   (i) SEQUENCE CHARACTERISTICS:
      (A) LENGTH: 349 amino acids
      (B) TYPE: amino acid
      (D) TOPOLOGY: linear
   (ii) MOLECULE TYPE: protein
   (xi) SEQUENCE DESCRIPTION: SEQ ID NO: 18:
   Met Gln Gln Asp Gly Leu Ser Ser Val Asn Gln Leu Gly Gly Leu Phe
   1 5 10 15
   Val Asn Gly Arg Pro Leu Pro Leu Asp Thr Arg Gln Gln Ile Val Gln
   20 25 30
   Leu Ala Ile Arg Gly Met Arg Pro Cys Asp Ile Ser Arg Ser Leu Lys
   35 40 45
   Val Ser Asn Gly Cys Val Ser Lys Ile Leu Gly Arg Tyr Tyr Arg Thr
   50 55 60
   Gly Val Leu Glu Pro Lys Cys Ile Gly Gly Ser Lys Pro Arg Leu Ala
   65 70 75 80
   Thr Pro Ala Val Val Ala Arg Ile Ala Gln Leu Lys Asp Glu Tyr Pro
   85 90 95
   Ala Leu Phe Ala Trp Glu Ile Gln His Gln Leu Cys Thr Glu Gly Leu
   100 105 110
   Cys Thr Gln Asp Lys Ala Pro Ser Val Ser Ser Ile Asn Arg Val Leu
   115 120 125
   Arg Ala Leu Gln Glu Asp Gln Ser Leu His Trp Thr Gln Leu Arg Ser
   130 135 140
   Pro Ala Val Leu Ala Pro Val Leu Pro Ser Pro His Ser Asn Cys Gly
   145 150 155 160
   Ala Pro Arg Gly Pro His Pro Gly Thr Ser His Arg Asn Arg Ala Ile
   165 170 175
   Phe Ser Pro Gly Gln Ala Glu Ala Leu Glu Lys Glu Phe Gln Arg Gly
   180 185 190
   Gln Tyr Pro Asp Ser Val Ala Arg Gly Lys Leu Ala Ala Ala Thr Ser
   195 200 205
   Leu Pro Glu Asp Thr Val Arg Val Trp Phe Ser Asn Arg Arg Ala Lys
   210 215 220
   Trp Arg Arg Gln Glu Lys Leu Lys Trp Glu Ala Gln Leu Pro Gly Ala
   225 230 235 240
   Ser Gln Asp Leu Thr Val Pro Lys Asn Ser Pro Gly Ile Ile Ser Ala
   245 250 255
   Gln Gln Ser Pro Gly Ser Val Pro Ser Ala Ala Leu Pro Val Leu Glu
   260 265 270
   Pro Leu Ser Pro Ser Phe Cys Gln Leu Cys Cys Gly Thr Ala Pro Gly
   275 280 285
   Arg Cys Ser Ser Asp Thr Ser Ser Gln Ala Tyr Leu Gln Pro Tyr Trp
   290 295 300
   Asp Cys Gln Ser Leu Leu Pro Val Ala Ser Ser Ser Tyr Val Glu Phe
   305 310 315 320
   Ala Trp Pro Cys Leu Thr Thr His Pro Val His His Leu Ile Gly Gly
   325 330 335
   Pro Gly Gln Val Pro Ser Thr His Cys Ser Asn Trp Pro
   340 345